# EUROPEAN PATENT APPLICATION

(11) **EP 4 825 880 A1**
(43) Date of publication of application: **21.10.2026**
(21) Application number: 26163762.3
(22) Date of filing: 10.03.2026
(51) Int. Cl.: C08B 37/00, A61K 8/73, A61K 8/9728, A61Q 19/08, C08B 37/08, C08L 5/08

(54) **AN EXTRACT FROM T. FUCIFORMIS COMPRISING OR, ALTERNATIVELY, CONSISTING OF A HYALURONIC ACID OR A SALT THEREOF WITH HIGH MOLECULAR WEIGHT, A MIXTURE AND A COMPOSITION COMPRISING SAID EXTRACT, USES THEREOF, AND A NEW PROCESS FOR EXTRACTING HYALURONIC ACID WITH HIGH MOLECULAR WEIGHT FROM A FUNGUS**

(30) Priority: 15.04.2025 IT 202500008466
(71) Applicant: Vivatis Pharma GmbH, 20097 Hamburg (DE)
(72) Inventor: CERANA, Giorgio Stefano, 20097 Hamburg (DE); BOS, Peter, 20097 Hamburg (DE)
(74) Representative: Marben S.r.l.

(57) **Abstract**

The present invention relates to an extract from *T.fuciformis* which comprises or consists of polysaccharides from *T.fuciformis* in an amount by weight comprised from 80% to 95% by weight, with respect to the weight of the extract, wherein from 75% to 95% of said polysaccharides from *T.fuciformis* are polysaccharides identified as hyaluronic acid or a salt thereof having a weight average molecular weight comprised from 1000 kDa to 6000 kDa, to the therapeutic and non-therapeutic uses thereof, for example as an additive, excipient, or ingredient in the preparation of pharmaceuticals, medical devices, nutraceutical products, foods for special medical purposes (AFMS), food products or food supplements, and to the production process thereof.

## Description

The present invention relates to an extract from *T.fuciformis* and a mixture which comprises or, alternatively, consists of at least one glycosaminoglycan, obtained from a starting plant material, chosen from the group comprising or, alternatively, consisting of hyaluronic acid or a salt thereof with high molecular weight for example greater than 1000 kDa (a salt of the hyaluronate anion) (in short, together or separately, referred to as HA) of plant origin with a high degree of purity and reduced content of contaminants and/or by-products. The extract from *T.fuciformis* or the mixture comprises or consists of polysaccharides from *T.fuciformis* in an amount by weight comprised from 80% to 95% by weight, with respect to the weight of the extract, wherein 75% to 95% of said polysaccharides from *T.fuciformis* are polysaccharides identified as hyaluronic acid or a salt thereof having a weight average molecular weight comprised from 1000 kDa to 6000 kDa.

Furthermore, the present invention relates to a use of the aforementioned mixture as an additive, excipient, or ingredient in the preparation of pharmaceuticals, medical devices, nutraceutical products, foods for special medical purposes (AFMS), food products or food supplements.

Furthermore, the present invention relates to a composition comprising (i) said mixture comprising or, alternatively, consisting of hyaluronic acid or a salt thereof (a salt of the hyaluronate anion) (in short, together or separately, referred to as HA), of plant origin, and (ii) optionally technological additives and excipients of pharmaceutical or food grade.

Furthermore, the present invention relates to said composition comprising said mixture for use as a medicament. Furthermore, the present invention relates to said mixture and said composition for a non-therapeutic use (non-medical use) and, therefore, for a cosmetic or dermo-cosmetic use as a product for oral use, for example as a food supplement, or for topical use, for example as a cream or hydrogel or gel.

Furthermore, the present invention relates to said mixture and said composition for non-therapeutic use (non-medical use) and, therefore, for a cosmetic or dermo-cosmetic use as a product for oral use, for example as a food supplement, or for topical use, for example as a cream or hydrogel or gel, for counteracting skin ageing.

Furthermore, the present invention relates to said mixture and said composition comprising said mixture for use in a method of treatment, preventive or curative, for human beings and animals having specific ailments or disorders or pathologies, chosen from arthritis, osteoarthritis, arthrosis, joint pain, inflammation of the limbs and joints, gastroesophageal reflux, inflammatory skin diseases.

Furthermore, the present invention relates to said mixture and to said composition for use in a method of preventive and/or curative treatment for human beings and animals having diseases and/or disorders and/or associated symptoms, wherein at least one of the following health benefits is necessary and/or advantageous: antioxidant effects, anti-inflammatory effects, beneficial effects on joints, cartilage, skin and epidermal thickness.

Also, the present invention relates to a process for preparing said mixture, and said composition comprising said mixture, which comprises or, alternatively, consists of hyaluronic acid or a salt thereof (a salt of the hyaluronate anion) (in short, together or separately, referred to as HA) of plant origin.

Finally, the present invention relates to the use of a fungus, from *T. fuciformis,* as a starting plant material for preparing a hyaluronic acid, or a salt thereof, of plant origin with a high degree of purity and a reduced content of contaminants and/or by-products.

Hyaluronic acid (HA) is a linear, non-sulphate glycosaminoglycan composed of repeating disaccharide units of D-glucuronic acid and N-acetyl-D-glucosamine, linked by alternating β-(1→3) and β-(1→4) glycosidic bonds. Hyaluronic acid (HA) is a ubiquitous component of the extracellular matrix (ECM), which contributes to tissue hydration, elasticity, and structural organization, while regulating cell proliferation, migration, and differentiation through receptors such as the multifunctional transmembrane glycoprotein CD44, i.e., the primary receptor for hyaluronic acid (HA) which is expressed in various isoforms in almost every type of human cell and which influences numerous intracellular signalling pathways that regulate cellular processes.

*Tremella fuciformis (T. fuciformis),* an edible medicinal fungus, is commonly known as snow fungus, snow ear fungus, silver ear fungus, and white jelly fungus. It is called *"Yiner"* or *"Baimuer"* in China, it belongs to the order of *Tremellales* and the family of *Tremellaceae,* and has been traditionally used for promoting health and longevity in China and other East Asian countries for many years. Many bioactive substances have been discovered in T. *fuciformis,* including fatty acids, proteins, enzymes, polysaccharides, phenols, flavonoids, dietary fibres and trace elements.

Among these, polysaccharide has been identified as one of the main bioactive components of *T. fuciformis,* widely present in fruiting bodies, spores, mycelia and fermentation liquor. The chemical structure of *T. fuciformis* polysaccharides consists of a linear chain of (1→3) α-D-mannane with side chains composed of glucuronic acid, xylose and fucose; for example, the *T. fuciformis* polysaccharides may have a chemical structure consisting of a linear chain (1→3) of α-D-mannose with highly branched side chains of β-D-xylose, α-D-fucose and β-D-glucuronic acid.

Under various experimental conditions, it is possible to collect different polysaccharide fractions, by isolating different molecular weights going from 5.82 × 10⁵ Da to 3.74 × 10⁶ Da. However, the beneficial properties depend on the composition, which influences the molecular weight of the polysaccharides, varying from 8x10³ to 6x10⁶ Da based on the different mixes of fucose, galactose, xylose, mannose, glucose and glucuronic acid obtained from extraction methods. The extraction methods are the most diverse and are able to influence factors such as the content, quality and physicochemical characteristics, such as for example the chemical and physical composition of the polysaccharides; therefore, the choice of the extraction method depends on the possible application of the extract. For example, hot water extraction, alkali extraction, sonication-assisted extraction, cold water extraction and others are known. For example, the fruit of *T. fuciformis,* by extraction in hot water, was found to be composed of (1→2)- and (1→4) -bound mannose and (1→3)-bound glucans or in the mycelium mannose, rhamnose, glucuronic acid, glucose in a different ratio. In addition, *T. fuciformis* is composed of xylose, mannose, and glucuronic acid linked by an α-1,3-glycosidic bond, with side chains composed of galactose, arabinose, and small amounts of fucose. Based on this composition, *T. fuciformis* can be used as a moisturizing additive in the cosmetic industry.

Moreover, an analysis of the fruiting body of these fungi showed that it contains 6.7-10.0% of protein, 65.0-71.2% of carbohydrates, 0.6-12.8% of fats, 2.4-2.7% of crude fibre, 4.0-5.4% of inorganic salts, 15.2-18.7% of water and a small amount of B vitamins. Finally, the fruiting body of *T.fuciformis* contains many uronic acids (the main part is glucuronic acid) and hydroxyls, which are important for stabilizing the extract for a long time without significant changes in its physical and chemical properties, such as electrical conductivity or pH.

The *T. fuciformis* polysaccharides can be classified into the following categories: acidic polysaccharides, neutral polysaccharides, oligosaccharide acids, and cell wall polysaccharides. Research on the structure-activity relationship of the active polysaccharides has demonstrated that the viscosity of the polysaccharide has a great influence on its active function in the body. The rheological study of *T. fuciformis* not only has important significance for science, but also for food, medicine, cosmetics and other correlated industries.

Therefore, it would be desirable to be able to have a hyaluronic acid and/or a salt thereof of plant origin with a repeatable composition, reproducible in a standardized manner on an industrial scale, both in terms of process and chemical-physical characteristics and with a high weight average molecular weight, preferably with a weight average molecular weight greater than 1000 kDa, more preferably greater than 1500 kDa.

Some extraction processes are known.

Alcohol precipitation has been the predominant technique for isolating the polysaccharides from *T. fuciformis* (TFPS), although this approach is characterized by prolonged extraction duration and significant polysaccharide depletion. Furthermore, the polysaccharides obtained are typically contaminated by various impurities, including pigments, proteins and nucleic acids, which require subsequent purification processes. The common purification processes may include ion exchange chromatography, to separate neutral and acidic polysaccharides with elution (carried out using water and gradients containing different concentrations of sodium chloride), or gel filtration chromatography, to separate the polysaccharides according to their molecular weight. In addition, an ultrasound-assisted ethanol precipitation method has also been developed, but it still has many limitations and drawbacks. Therefore, it is necessary to have a process for preparing HA that is repeatable, with low production times and capable of providing a HA and/or a salt thereof with high molecular weight and excellent quality.

As mentioned, hyaluronic acid is a non-sulphate anionic glycosaminoglycan (GAG) that is abundantly distributed in the connective, epithelial, and neural tissues of vertebrates. The hyaluronic acid performs important structural, rheological and physiological functions.

Despite the numerous and undisputed - in terms of effectiveness - medical applications of hyaluronic acid in mammals, in particular in human beings, the preparation processes by extraction from animal precursors are today faced with growing concerns and fears, including of an ethical, religious and moral nature. The greatest concerns and fears derive from the use of animal-derived or animal-origin products for the preparation of a hyaluronic acid or a sulphate salt thereof, especially when these compounds or salts are intended for nutritional, biomedical or pharmaceutical applications.

Therefore, in the field of pharmaceuticals, medical devices, nutraceutical products, foods for special medical purposes (AFMS), food supplements or food products, and cosmetic products, the need and necessity is strongly felt by market operators to be able to have a hyaluronic acid or a salt thereof, which are prepared in an alternative way to what is already known and which are available to all categories of consumers including vegans, vegetarians, people suffering from allergies and all those to whom - for religious or ideological reasons - access to products or medicines containing hyaluronic acid or a salt thereof is currently precluded. In addition, there is a need to produce hyaluronic acid of non-animal origin with economically advantageous processes compared to what is known in the art and easy to apply.

The Applicant, after a long and intense research and development activity, has developed a technology and a preparation process able to provide an adequate response to the existing limits, drawbacks and problems.

Therefore, object of the present invention is an extract from *T.fuciformis* and a mixture preferably in the form of powder and/or granules that comprises it, which comprises or, alternatively, consists of at least one glycosaminoglycan, obtained from a starting plant material, selected from the group comprising or, alternatively, consisting of hyaluronic acid or a salt thereof with high weight average molecular weight, for example greater than 1000 kDa (a salt of the hyaluronate anion) (in short, together or separately, referred to as HA), having the characteristics as defined in the appended claims.

Preferably the extract from *T.fuciformis,* comprises or, alternatively, consists of polysaccharides from *T.fuciformis* in an amount by weight comprised from 80% to 95% by weight, with respect to the total weight of the extract, wherein from 75% to 95% of said polysaccharides from *T.fuciformis* are polysaccharides identified as hyaluronic acid or a salt thereof having a weight average molecular weight comprised from 1000 kDa to 6000 kDa. Preferably, the extract from *T.fuciformis* and the mixture comprise or, alternatively, consist of polysaccharides *T. fuciformis* having a weight average molecular weight comprised from 1000 kDa to 6000 kDa, wherein at least 70% of said polysaccharides from *T. fuciformis* having a weight average molecular weight comprised from 1000 kDa to 6000 kDa, is represented by hyaluronic acid from *T. fuciformis;* preferably, said at least 70% by weight is represented by hyaluronic acid having a weight average molecular weight comprised from 1500 kDa to 5000 kDa. Said mixture may also optionally comprise technological additives and excipients of pharmaceutical or food grade. Furthermore, object of the present invention is a use of the aforementioned mixture as an additive, excipient, or ingredient in the preparation of pharmaceuticals, medical devices, nutraceutical products, foods for special medical purposes (AFMS), food products or food supplements, said use having the characteristics as defined in the appended claims.

Another object of the present invention is a composition comprising (i) said mixture comprising or, alternatively, consisting of hyaluronic acid or a salt thereof (a salt of the hyaluronate anion) (in short, together or separately, referred to as HA), having the characteristics as defined in the appended claims.

Another object of the present invention is a mixture and at least one technological additive or excipient, or a composition for use as a medicament (first medical use), having the characteristics as defined in the appended claims.

Another object of the present invention is a mixture and a composition, containing said mixture, for a non-therapeutic use (non-medical use) and, therefore, for cosmetic or dermo-cosmetic use as a product for oral use, for example as a food supplement, or for topical use, for example as a cream or hydrogel or gel, having the characteristics as defined in the appended claims.

Another object of the present invention is the mixture or a composition comprising the mixture for a non-therapeutic use (non-medical use) and, therefore, for cosmetic or dermo-cosmetic use as a product for oral use, for example as a food supplement, or for topical use, for example as a cream or hydrogel or gel, for counteracting skin ageing having the characteristics as defined in the appended claims.

Another object of the present invention is a mixture or a composition comprising said mixture for use in a method of treatment, preventive or curative, for human beings or animals, having specific ailments or disorders or pathologies, chosen from arthritis, osteoarthritis, arthrosis, joint pain, inflammation of the limbs and joints, gastroesophageal reflux (second medical use), inflammation of the cartilage, skin and epidermis, said use having the characteristics as defined in the appended claims.

Furthermore, another object of the present invention is a process for preparing said mixture, or said composition comprising said mixture, which comprises or, alternatively, consists of hyaluronic acid or a salt thereof (a salt of the hyaluronate anion) (in short, together or separately, referred to as HA), of plant origin, having the characteristics as defined in the appended claims.

In the context of the present invention with plant HA from *T. fuciformis,* or non-animal HA or Greenluronic^{®} reference is always made to HA obtained from *T.fuciformis* subject-matter of the present invention.

Finally, object of the present invention is a use of a fungus, from *T. fuciformis,* as a starting plant material for preparing a hyaluronic acid or a salt thereof, with a high degree of purity and a reduced content of contaminants and/or by-products, having the characteristics as defined in the appended claims.

Preferred embodiments of the present invention will now be described below, with the aid of the accompanying tables, without wishing in any way to limit the scope of the present invention, wherein:
- figure 1 and 1A refers to a Flow Chart of production of plant HA from *Tremella fuciformis* with high molecular weight;
- figure 2 refers to an analysis of the molecular weight of the non-animal hyaluronic acid. In (A), an example of 1% agarose gel. The loads of the samples are described by the following abbreviations: L1 *= HiLadder* at a specific standard molecular weight for HA detection; L2 = lane loaded with 3 mg/ml of Published Reference; L3 = lane loaded with 3 mg/ml of the non-animal hyaluronic acid extract. In (B), the chromatogram is obtained from the particle size distribution.
- figure 3 refers to a HPLC-UV and HRMS analysis of the non-animal hyaluronic acid after enzymatic hydrolysis with chondroitinase AC. In (A), HPLC-UV chromatogram of a sample of the non-animal hyaluronic acid and relative positive HRMS spectrum. In (B), HPLC-UV chromatograms of a mixture of standard chondroitin disaccharides ΔDi-0S, ΔDi-4S and ΔDi-6S and in (C), a solution of the standard disaccharide ΔDi-HA of HA.
- figure 4 refers to the 13C CPMAS NMR spectrum of the non-animal hyaluronic acid recorded using a MAS frequency of 11 kHz and a contact time CP of 0.5 ms. The image (on the right) shows the 1H NMR MAS spectrum of the non-animal hyaluronic acid recorded with a MAS frequency of 15 kHz. The insert (on the left) shows the molecular structure of the hyaluronic acid.
- figure 5 refers to ATR-IR spectrum of the non-animal hyaluronic acid. (curve a) and standard hyaluronic acid (curve b).
- figure 6 refers to representative MALDI-TOF mass spectra in the region 300 - 5,000 m/z. The abbreviations comprise: YET = non-animal hyaluronic acid extract before extraction; YEF = non-animal hyaluronic acid intermediate extract; non-animal HA = non-animal hyaluronic acid.
- figure 7 refers to an example of determination of the molecular weight of GREENIURONIC^{®} (non-animal, or plant hyaluronic acid from *T.fuciformis*) on 1% agarose gel. In (A), the loads of the samples are described by the following abbreviations: L1 = at standard molecular weight *HiLadder* specific for HA detection; L2 = lane loaded with 3 mg/ml of Published Reference; L3 = lane loaded with 3 mg/ml of the non-animal hyaluronic acid extract. In (B), the loads of the samples are described by the following abbreviations: L1 = standard molecular weight Mega + *HiLadder* specific for HA detection; L2= lane loaded with 1mg/ml of pure Greenluronic^{®} (non-animal, or plant hyaluronic acid from *T.fuciformis),* L3 = lane loaded with 1mg/ml of Greenluronic^{®} prepared in 1% SDS w/v; L4= lane loaded with 1mg/ml of Greenluronic^{®} prepared in 3% SDS w/v; L5= lane loaded with 1mg/ml of Greenluronic^{®} prepared in 5% SDS w/v; lane loaded with 1mg/ml of Greenluronic^{®} prepared in 8% SDS w/v.
- figure 8 refers to NMR C CPMAS spectrum of 13Greenluronic^{®} (non-animal hyaluronic acid) recorded using a MAS speed of 11 kHz and a contact time CP of 0.5 ms. In (A), NMR spectrum of Greenluronic^{®} and in (B), NMR spectrum of Greenluronic^{®} with reduced molecular weight.
- figure 9 refers to a schematic experimental protocol illustrated in the present patent application.
- Figure 10 refers to the biological analysis of the non-animal hyaluronic acid and of the sodium hyaluronate in gastric cells (GTL-16). In (A) cell viability analysis from 1 to 4 hours measured by MTT; in (B) integrity analysis through TEER value using EVOM3; in (C) ROS production measured by cytochrome C reduction from 1 to 4 hours; in (D) TNFα production analysis after 4 hours by specific ELISA kit; in (E) CD44 analysis after 4 hours by western blot; in (F) HA quantification at intracellular and extracellular level after 4 hours by specific ELISA kit. The data are the mean ± SD of five independent experiments performed in triplicate with respect to the control values (0% line). * p<0.05 vs. control; # p<0.05 vs. sodium hyaluronate
- figure 11 refers to an example of determination of the molecular weight of hyaluronic acid on 1% agarose gel. The loads of the samples are described by the following abbreviations: MW = standard molecular weight Mega + *HiLadder* specific for hyaluronic acid detection; L1 = standard molecular weight Mega + *HiLadder,* L3 = lane loaded with 10 µL of the non-animal hyaluronic acid; L3 = lane loaded with 10 µL of sodium hyaluronate.
- figure 12 refers to the biological analysis of the non-animal hyaluronic acid and of the sodium hyaluronate in intestinal cells (Caco-2). In (A) cell viability analysis from 2 to 6 hours measured by MTT; in (B) integrity analysis by TEER value using EVOM3; in (C) ROS production measured by cytochrome C reduction from 2 to 6 hours; in (D) TNFα production analysis after 6 hours by specific ELISA kit; in (E) CD44 analysis after 6 hours by western blot; in (F) hyaluronic acid quantification at intracellular and extracellular level after 6 hours by specific ELISA kit; and in (G), hyaluronidase levels measured by ELISA kit. The data are the mean ± SD of five independent experiments performed in triplicate with respect to the control values (0% line). * p<0.05 vs. control; # p<0.05 vs. sodium hyaluronate
- figure 13 refers to an example of determination of the molecular weight of hyaluronic acid on 1% agarose gel. The loads of the samples are described by the following abbreviations: MW = standard molecular weight Mega + *HiLadder* specific for hyaluronic acid detection; L1 = standard molecular weight Mega + *HiLadder,* L3 = lane loaded with 10 µL of the non-animal hyaluronic acid; L3 = lane loaded with 10 µL of sodium hyaluronate.
- figure 14 refers to the biological analysis of the non-animal hyaluronic acid and of the sodium hyaluronate in hepatic cells (HepG2). In (A), cell viability analysis was measured by MTT; in (B), TEER value measurement before and after 24-hour treatment; in (C), ROS production was measured by cytochrome C reduction; in (D), TNFα production analysis by specific ELISA kit; in (E), CD44 analysis was measured by western blot; in (F), hyaluronic acid quantification at intracellular and extracellular level by specific ELISA kit; and in (G), hyaluronidase levels measured by ELISA kit. The data represent the mean ± SD of five independent experiments performed in triplicate with respect to the control values (0% line). * p<0.05 vs. control; # p<0.05 vs. sodium hyaluronate.
- figure 15 refers to an example of determination of the molecular weight of hyaluronic acid on 1% agarose gel. The loads of the samples are described by the following abbreviations: MW = standard molecular weight Mega + *HiLadder* specific for HA detection; L1 = standard molecular weight Mega + *HiLadder,* L3 = lane loaded with 10 µL of the non-animal hyaluronic acid; L3 = lane loaded with 10 µL of sodium hyaluronate.
- figure 16 refers to the biological analysis of the non-animal hyaluronic acid and of the sodium hyaluronate on renal cells (HEK293). In (A), cell viability analysis was measured by MTT; in (B), ROS production was measured by cytochrome C reduction; in (C), TNFα production analysis by specific ELISA kit; in (D), CD44 analysis was performed by western blot; in (E), hyaluronic acid quantification at intracellular and extracellular levels by specific ELISA kit; and in (F), hyaluronidase levels measured by ELISA kit. The data represent the mean ± SD of five independent experiments performed in triplicate with respect to the control values (0% line). * p<0.05 vs. control; # p<0.05 vs. sodium hyaluronate.
- figure 17 refers to an example of determination of the molecular weight of hyaluronic acid on 1% agarose gel. The loads of the samples are described by the following abbreviations: MW = standard molecular weight Mega + *HiLadder* specific for HA detection; L1 = standard molecular weight Mega + *HiLadder,* L3 = lane loaded with 10 µL of non-animal HA; L3 = lane loaded with 10 µL of sodium hyaluronate.
- figure 18 refers to the biological analysis of the non-animal hyaluronic acid and the sodium hyaluronate in chondrocytes (T/C28a2). In (A), cell viability analysis measured by MTT; in (B), ROS production measured by cytochrome C reduction; in (C), analysis of TNFα production by specific ELISA kit; in (D), CD44 analysis by western blot; in (E), quantification of hyaluronic acid at intracellular and extracellular levels by specific ELISA kit. The data are the mean ± SD of five independent experiments performed in triplicate with respect to the control values (0% line). * p<0.05 vs. control; # p<0.05 vs. sodium hyaluronate.
- figure 19 refers to an example of determination of the molecular weight of hyaluronic acid on 1% agarose gel. The loads of the samples are described by the following abbreviations: MW = standard molecular weight Mega + *HiLadder* specific for HA detection; L1 = standard molecular weight Mega + *HiLadder,* L3 = lane loaded with 10 µL of non-animal HA; L3 = lane loaded with 10 µL of sodium hyaluronate.
- figure 20 refers to the biological analysis of non-animal HA and of the sodium hyaluronate at the keratinocyte level. In (A), cell viability analysis measured by MTT; in (B), ROS production measured by cytochrome C reduction; in (C), analysis of TNFα production by specific ELISA kit; in (D), CD44 analysis by western blot; in (E), quantification of hyaluronic acid at intracellular and extracellular levels by specific ELISA kit. The data are the mean ± SD of five independent experiments performed in triplicate with respect to the control values (0% line). * p<0.05 vs. control; # p<0.05 vs. sodium hyaluronate.
- figure 21 refers to an example of determination of the molecular weight of hyaluronic acid on 1% agarose gel. The loads of the samples are described by the following abbreviations: MW = standard molecular weight Mega + *HiLadder* specific for hyaluronic acid; L1 = standard molecular weight Mega + *HiLadder,* L3 = lane loaded with 10 µL of non-animal HA; L3 = lane loaded with 10 µL of sodium hyaluronate.

It should be noted that, within the present description, the expression "HA" indicates hyaluronic acid and/or a salt thereof, or a hyaluronate, or combinations thereof, also having a weight average molecular weight equal to or different from each other.

It should be noted that, in this description, the expressions "starting plant material" and "starting material of plant origin" are synonymous with each other, and are therefore used interchangeably.

### DETAILED DESCRIPTION OF THE INVENTION

An object of the present invention is a mixture comprising or, alternatively, consisting of at least one glycosaminoglycan obtained from a starting material of plant origin. Said material of plant origin is selected from the group comprising or, alternatively, consisting of one or more natural fungi.

Said glycosaminoglycan is selected from the group comprising or, alternatively, consisting of a hyaluronic acid or a salt thereof, and/or a hyaluronate anion (in short, HA).

The starting plant material is a fungus. The fungus is a fungus that grows and is found in nature, for example it can be found and harvested in the woods, but it can also be grown in greenhouses.

The fungus belongs to the sub-kingdom *Dikarya,* preferably it is of the division *Basidiomycota.*

Dikarya is a subdomain of fungi which comprises the divisions *Ascomycota* and *Basidiomycota.* Basidiomycetes (*Basidiomycota* R.T. Moore, 1980) is one of the largest *phyla* that make up the fungus kingdom.

The starting plant material is a fungus of the species *Tremella fuciformis* or said starting material of plant origin comprises or, alternatively, consists of a fungus of the species *Tremella fuciformis.* For example, it is a *Tremella fuciformis Berk.*

*Tremella fuciformis* (Berk. 1856) (also known as snow fungus, or silver ear fungus) is a fungus native to tropical and subtropical areas, where it thrives on the dead trunks of broadleaves, and which is also cultivated to cope with intensive consumption - especially in Japan and China - in cooking and traditional medicine. *Tremella fuciformis* produces fruiting bodies (basidiocarps) that are gelatinous, white in colour, similar to fronds.

The use of *Tremella fuciformis* in the present invention is particularly advantageous in that, starting from this starting plant material, it is possible, with the extraction technology developed herein, to produce HA completely of plant origin, with high weight average molecular weight, for example greater than 1000 kDa. The glycosaminoglycan HA contained in the mixture obtained by the process of the present invention has particular characteristics that make it particularly effective.

By means of the extraction process of the present invention a liquid extract from *T.fuciformis* is obtained, in particular an extract enriched in polysaccharides from *T.fuciformis* that contains at least 75% by weight, preferably from 80% to 95% by weight, of said polysaccharides from *T.fuciformis,* with respect to the weight of the enriched extract. At least 70%, preferably from 75% to 95%, of said polysaccharides from *T.fuciformis* are identified as HA. The polysaccharides from *T.fuciformis,* obtained by the process of the present invention have a weight average molecular weight comprised from 1000 kDa to 6000 kDa, preferably comprised from 1500 kDa to 5000 kDa, more preferably comprised from 2000 kDa to 4000 kDa, for example a weight average molecular weight comprised from 1500 kDa to 4000 kDa, or about 2500 kDa, or 3000 kDa, or 3500 kDa, or 4500 kDa, or 5500 kDa. More precisely, the hyaluronic acid or salt thereof (hyaluronate), i.e., the polysaccharides from *T.fuciformis* identified as HA, obtained by the process of the present invention has a weight average molecular weight comprised from 1000 kDa to 6000 kDa, preferably comprised from 1500 kDa to 5000 kDa, even more preferably comprised from 2000 kDa to 4000 kDa, for example a weight average molecular weight comprised from 1500 kDa to 4000 kDa, or about 2500 kDa, or 3000 kDa, or 3500 kDa, or 4500 kDa, or 5500 kDa. The extraction process of the present invention allows to obtain an extract enriched in polysaccharides from *T.fuciformis* that contains at least 75% by weight, preferably from 80% to 95% by weight, more preferably from 85% to 90% by weight, of polysaccharides from *T.fuciformis,* with respect to the total weight of the enriched extract, wherein said polysaccharides from *T.fuciformis* have a weight average molecular weight comprised from 1000 kDa to 6000 kDa, preferably comprised from 1500 kDa to 5000 kDa, more preferably comprised from 2000 kDa to 4000 kDa, for example a weight average molecular weight comprised from 1500 kDa to 4000 kDa, or about 2500 kDa, or 3000 kDa, or 3500 kDa, or 4500 kDa, or 5500 kDa. At least 70%, preferably from 75% to 95%, more preferably from 80% to 90%, of said polysaccharides from *T*. *fuciformis* are polysaccharides identified as HA having a weight average molecular weight comprised from 1000 kDa to 6000 kDa, preferably comprised from 1500 kDa to 5000 kDa, more preferably comprised from 2000 kDa to 4000 kDa, for example a weight average molecular weight comprised from 1500 kDa to 4000 kDa, or about 2500 kDa, or 3000 kDa, or 3500 kDa, or 4500 kDa, or 5500 kDa. Thus, with the process of the present invention an extract from *T.fuciformis* is obtained which comprises or consists of polysaccharides from *T.fuciformis* in an amount by weight comprised from 80% to 95% by weight, with respect to the weight of the extract, wherein from 75% to 95% of said polysaccharides from *T.fuciformis* are polysaccharides identified as hyaluronic acid or a salt thereof having a weight average molecular weight comprised from 1000 kDa to 6000 kDa.

Preferably, with the process of the present invention a product or a mixture (preferably in the form of powder and/or granules) or an enriched extract is obtained that contains about 90% by weight of *Tremella fuciformis* polysaccharides and of them beyond 85%, is represented by polysaccharides identified as HA and/or a salt thereof having a weight average molecular weight comprised from 1500 kDa to 4000 kDa, for example greater than 2000 kDa, as reported in the experimental part.

Preferably, with the process of the present invention a product or a mixture (preferably in the form of powder and/or granules) or an extract is obtained that contains, in addition to the polysaccharides from *T.fuciformis* and, among them those identified as HA, also uronic acid in an amount by weight comprised from 1% to less than 3%, preferably comprised from 1.5% to 2.5%. This indicates an advantageous yield of the extraction process. In particular, the extraction process of the present invention provides for a low temperature drying step, carried out for example by lyophilization, which maintains a uronic acid value comprised from 2% to 2.8% by weight, with respect to the total weight of the enriched extract.

Preferably, with the process of the present invention a product or a mixture (preferably in the form of powder and/or granules) or an enriched extract is obtained that contains, in addition to the polysaccharides from *T.fuciformis* and, among them those identified as HA, and, optionally in addition to said uronic acid in an amount by weight comprised from 1% to less than 3%, preferably comprised from 1.5% to 2.5%, also an amount by weight of NAG comprised from 0.01% to less than 0.1%, preferably comprised from 0.04% to 0.08% by weight, with respect to the total weight of the enriched extract.

Preferably, with the process of the present invention a product or a mixture (preferably in the form of powder and/or granules) or an enriched extract is obtained that contains, in addition to the polysaccharides from *T.fuciformis* and, among them those identified as HA, and, optionally, in addition to said uronic acid in an amount by weight comprised from 1% to less than 3%, preferably comprised from 1.5% to 2.5% and, optionally, in addition to said NAG in an amount by weight comprised from 0.01% to less than 0.1%, preferably comprised from 0.04% to 0.08%, also water in an amount by weight comprised from 2% to 8%, preferably comprised from 4% to 6% by weight, with respect to the total weight of the enriched extract.

Therefore, the extract, the product or the mixture (preferably in the form of powder and/or granules) obtained or obtainable by the process of the present invention comprises total polysaccharides from *T. fuciformis* in an amount by weight comprised from 80% to 95% by weight, preferably from 85% to 90% by weight, for example equal to 90.6% by weight, with respect to the total weight of the enriched extract, of which from 75% to 95%, preferably from 80% to 90%, for example 87.8%, of said total polysaccharides *T.fuciformis* are polysaccharides identified as HA having a weight average molecular weight comprised from 1000kDa to 6000 kDa, preferably from 1500 kDa to 5000 kDa, more preferably comprised from 2000 kDa to 4000 kDa, wherein the extract, the product or the mixture (preferably in the form of powder and/or granules) obtained or obtainable by the process of the present invention further comprises:
- uronic acid in an amount by weight comprised from 1% to less than 3%, preferably comprised from 1.5% to 2.5%, for example 2.39 by weight, with respect to the total weight of the enriched extract; and/or
- NAG in an amount by weight comprised from 0.01% to less than 0.1%, preferably comprised from 0.04% to 0.08% by weight, with respect to the total weight of the enriched extract; and/or
- water in an amount by weight comprised from 2% to 8%, preferably comprised from 4% to 6% by weight, with respect to the total weight of the enriched extract, for example 7.6% by weight, with respect to the total weight of the enriched extract.

For example, the extract, the product or the mixture (preferably in the form of powder and/or granules) obtained or obtainable by the process of the present invention comprises one of said uronic acid, NAG, water (in the respective amount indicated above). For example, the extract, the product or the mixture (preferably in the form of powder and/or granules) obtained or obtainable by the process of the present invention comprises two components selected from said uronic acid, NAG, water (in the respective amounts indicated above). For example, the extract, the product or the mixture (preferably in the form of powder and/or granules) obtained or obtainable by the process of the present invention comprises all three said uronic acid, NAG, water (in the respective amounts indicated above). The reduced presence of NAG, as well as the relatively low content of uronic acid, as well as the low water content, indicates a good efficiency of the extraction process that allows to obtain an extract enriched in polysaccharides identified as HA which are able to maintain a structural stability and their molecular weight in the gastric, intestinal, hepatic and renal environment, thus maintaining a high bioavailability and bioactivity at the level of the chondrocytes and keratinocytes and therefore an antioxidant and anti-inflammatory activity such as to allow the therapeutic or non-therapeutic use thereof to treat joints, cartilage, skin and thickness of the epidermis.

One gram of the enriched extract obtained by the process, added to 100 ml of ultrapure water at room temperature (25 °C) and under continuous stirring, has a pH comprised from 5.5 to 7.5, preferably comprised from 6 to 7, for example equal to 6.5.

The pH greatly influences not only the functional properties of the polysaccharides identified as HA contained in the enriched extract (such as, for example, its colour, flavour, and texture) but also the ability to maintain their structural integrity. In particular, to maintain their structural integrity, the polysaccharides identified as HA contained in the enriched extract must have a pH greater than 4 and less than 11 when dissolved in solution, otherwise they could undergo degradation. Said functional properties and the ability to maintain structural integrity are both of fundamental importance for the therapeutic or non-therapeutic use of the enriched extract.

The object of the present invention is a mixture which comprises or, alternatively, consists of the extract obtained or obtainable by the process of the present invention.

Preferably the mixture is in the form of powder and/or granules.

The object of the present invention is a composition which comprises:
- the mixture, in turn comprising or, alternatively, consisting of the extract obtained or obtainable by the process of the present invention;
- technological additives, and/or excipients, of pharmaceutical or food grade.

The composition, therefore, comprises the aforesaid mixture, which comprises or, alternatively, consists of a HA having a weight average molecular weight comprised from 1000 kDa to 6000 kDa, preferably comprised from 1500 kDa to 5000 kDa, even more preferably comprised from 2000 kDa to 4000 kDa, for example a weight average molecular weight of about 2500 kDa, or 3000 kDa, or 3500 kDa, or 4500 kDa, or 5500 kDa, and optionally technological additives and excipients of pharmaceutical or food grade.

Such a mixture and/or such a composition may be a pharmaceutical composition, a composition for medical device (EU) 2017/745, a composition with nutraceutical function, a composition for food for special medical purposes (AFMS), a composition for food supplement, or a composition for food product, or a composition for Novel food (EU) 2015/2283.

Such a mixture and/or such a composition is usable as a medicament, or as a composition for use in the treatment, preventive and/or curative, of arthritis, osteoarthritis, arthrosis, joint pain, inflammation of the limbs and joints, gastroesophageal reflux, inflammation of the cartilage, skin and epidermis.

The expression "skin inflammations" means chronic and non-chronic pathological conditions affecting the skin and of inflammatory origin such as, for example, chosen from the group comprising or, alternatively, consisting of: dermatitis, eczema, acne, erythema.

Preferably, the mixture and/or the composition are usable for use in the preventive and/or curative treatment of inflammatory skin diseases by oral administration at least once a day, and/or by topical administration to the skin at least once or twice a day.

Furthermore, said mixture and/or said composition find valid application for cosmetic or dermo-cosmetic use as a product for oral use, for example as a food supplement, or for topical use, for example as a cream or hydrogel or gel.

Moreover, said mixture and said composition find application for cosmetic or dermo-cosmetic use as a product for oral use, for example as a food supplement, or for topical use, for example as a cream or hydrogel or gel for counteracting skin ageing, preferably by oral administration at least once a day.

Object of the present invention is a technology and a process for preparing hyaluronic acid or hyaluronate (HA) , said process comprising at least one step of extracting hyaluronic acid or hyaluronate from a starting material of plant origin, for example a starting material of plant origin comprising or, alternatively, consisting of at least one natural fungus belonging to the sub-kingdom *Dikarya,* preferably division *Basidiomycota,* more preferably species *Tremella fuciformis.*

The process of the present invention does not comprise steps of fermentation and/or digestion with bacteria, such as for example the bacterial fermentations reported in patent documents WO 2012/152872 A1 and EP 2852437 B1 for the preparation of chondroitin or chondroitin sulphate.

The process subject-matter of the present invention comprises at least the following steps (Figure 1):
(i) a step of identifying and selecting one or more natural fungi as a starting plant material of a glycosaminoglycan (HA); for example, one or more natural fungi, fresh or in dry or dried form, preferably comprising or, alternatively, consisting of at least one fungus belonging to the subkingdom *Dikarya,* preferably division *Basidiomycota,* more preferably species *Tremella fuciformis;*
(ii) a step of carrying out a crushing or pulverisation of the starting plant material;
(iii) a step of extracting said glycosaminoglycan (HA) from the starting plant material obtained from step (ii) by using an extraction solvent, preferably an aqueous extraction solvent, even more preferably a water-only extraction solvent (e.g. distilled or bi-distilled water) to give an aqueous extract of said glycosaminoglycan (HA);
(iv) at least one step of enzymatic hydrolysis;
   - optionally, a step of inactivation of the enzymes used can be provided;
(v) at least one concentration step; preferably, at least one vacuum concentration step;
(vi) at least one precipitation step; preferably, at least one precipitation step conducted by using a precipitation solution comprising or, alternatively, consisting of at least one alcohol and/or water; preferably, the precipitation step may provide for the recovery of the alcohol, e.g., ethanol, and the addition of water in order to dilute the concentration of the alcohol present during the precipitation step and allow redissolution; preferably, the precipitation step may provide for at least one filtration or separation step; preferably, at least one filtration or separation step is preceded and/or followed by an addition of said precipitation solution comprising or, alternatively, consisting of at least one alcohol and/or water;
(vii) at least one step of removing said precipitation solution comprising or, alternatively, consisting of at least one alcohol and/or water;
   - optionally, at least one heat treatment step; preferably, at least one pasteurization heat treatment step;
(viii) a step of eliminating the water; preferably, a step of eliminating water by dehydration or lyophilization;
   - optionally, a sieving step, for example at 200 mesh, and packaging.

Unless otherwise indicated, the process steps of the present invention are conducted at a temperature comprised from 15 °C to 25 °C, preferably comprised from 18 °C to 22 °C and external pressure of 1 atmosphere.

In one version, the process subject-matter of the present invention comprises the following steps (Figure 1A):
(i) a step of identifying and selecting one or more natural fungi as a starting plant material of a glycosaminoglycan (HA); for example, one or more natural fungi, fresh or in dry or dried form, preferably comprising or, alternatively, consisting of at least one fungus belonging to the subkingdom *Dikarya,* preferably division *Basidiomycota,* more preferably species *Tremella fuciformis;*
(ii) optionally, a step of carrying out a crushing or pulverisation of the starting plant material;
(iii) a step of extracting said hyaluronic acid or salt thereof from the starting plant material identified and selected in said step (i) or obtained from step (ii) by using an extraction solvent comprising or consisting of water, to give an aqueous extract of said hyaluronic acid or a salt thereof;
(iv) at least one step of enzymatic hydrolysis of said aqueous extract to give a hydrolysed aqueous extract;
   - optionally, a step of inactivating the enzymes used in said hydrolysed aqueous extract may be provided;
(v) at least one concentration step; preferably, at least one step of vacuum concentration of said hydrolysed aqueous extract to give a concentrated aqueous extract;
(vi) at least one step of precipitation of said concentrated aqueous extract conducted by using a precipitation solution comprising or, alternatively, consisting of at least one alcohol, preferably ethanol, and/or water to give a precipitated aqueous extract (figure 1A, (a)); preferably, the precipitation step may provide for a sub-step of removing part of the alcohol, preferably ethanol, contained in the precipitation solution (recovery of the alcohol, e.g. ethanol - figure 1A, (b)), and/or may also provide preferably for a sub-step of diluting the concentration of the alcohol, preferably ethanol, contained in the precipitation solution by adding water to the concentrated aqueous extract, (figure 1A, (c)) in order to allow redissolution of the hyaluronic acid or salt thereof (figure 1A (d)); preferably the precipitation step may also provide for at least one filtration or separation step; preferably, it may also provide for at least one filtration or separation step preceded and/or followed by an addition of said precipitation solution comprising or, alternatively, consisting of at least one alcohol and/or water (not shown in figure 1A);
(vii) at least one step of removing, from said precipitated aqueous extract, said precipitation solution comprising or, alternatively, consisting of at least one alcohol and/or water to give an intermediate aqueous extract;
   (vii-1) at least one step of heat treatment, preferably pasteurization, of said intermediate aqueous extract to give an aqueous liquid extract;
(viii) optionally, a step of eliminating the water; preferably, a step of eliminating water by dehydration or lyophilization, to give an extract in powder and/or granules;
   - optionally, a step of sieving, for example at 200 mesh, and packaging the dry extract.

The extract subject-matter of the present invention (extract enriched in polysaccharides described above) is identified with the product of the heat treatment step (vii-1). The extract subject-matter of the present invention (extract enriched in polysaccharides described above) is therefore liquid.

By means of the step (viii) of eliminating the water a dry extract is obtained, for example in the form of powder and/or granules.

The mixture subject-matter of the present invention, and the composition containing said mixture, comprise, or, alternatively, consist of said dry extract, i.e., of the product of step (viii).

For example, the step (viii) of eliminating water, preferably by lyophilization, can be conducted, by way of example, by cooling the product obtained from step (vii) to a temperature comprised from -18 °C to -40 °C for a time comprised from 10 hours to 48 hours and subsequently placing the product under vacuum drying, for example at a pressure comprised from 10 mbar to 20 mbar.

The process subject-matter of the present invention has an optimal yield, since it advantageously allows to obtain an extract enriched in polysaccharides that contains, as mentioned, from 80% to 95% by weight of *T.fuciformis* polysaccharides, with respect to the weight of the extract.

The enriched extract is of high purity in terms of HA, in particular, the enriched extract obtained by the process contains polysaccharides identified as HA in an amount from 75% to 95%, with respect to the total of the polysaccharides contained in said enriched extract itself; the HA obtained has a high weight average molecular weight, i.e., comprised from 1000 kDa to 6000 kDa, preferably comprised from 1500 kDa to 5000 kDa, more preferably comprised from 2000 kDa to 4000 kDa.

To corroborate the above, the dry extract obtained by the process (after step (viii) of eliminating the water) contains HA detectable by HPLC analysis as the corresponding disaccharide ΔDi-HA generated by the enzymatic hydrolysis of chondroitinase AC of the enriched extract itself: this confirms both the presence of HA in the enriched extract, its high efficiency and yield of the extraction process.

Analysing, through analysis of the MALDI-TOF spectra (see the experimental part):
- the *Tremella fuciformis* powder,
- the extract at an intermediate step of the process, for example after the heat treatment step (vii-1), and
- the enriched extract obtained by the process of the present invention,
there is a progressive and marked reduction in the number of peaks from the *Tremella fuciformis* powder to the enriched extract obtained by the process of the present invention: this demonstrates that the process subject-matter of the present invention is remarkably efficient.

The advantageous yield and good efficiency of the extraction process can also be seen from:
- low content of uronic acid in the extract (an amount by weight comprised from 1% to less than 3%, preferably comprised from 1.5% to 2.5% by weight, with respect to the total weight of the enriched extract);
- low NAG content in the extract (an amount by weight comprised from 0.01% to less than 0.1%, preferably comprised from 0.04% to 0.08% by weight, with respect to the total weight of the enriched extract)
- low water content (an amount by weight comprised from 2% to 8%, preferably comprised from 4% to 6% by weight, with respect to the total weight of the enriched extract, for example 7.6% by weight, with respect to the total weight of the enriched extract).

The steps (vii) of removing the precipitation solution and (vii-1) of heat treatment (preferably pasteurization) allow obtaining a polysaccharide extract with properties suitable for its use as a natural substitute for hyaluronic acid.

Thanks to the process it is possible to obtain an extremely stable liquid extract (from the heat treatment step (vii-1)) and a dry extract (from the water elimination water (viii), in particular in biological environments such as the gastric environment, the intestinal environment, the hepatic environment and the renal environment. Stability is determined by the pH of the enriched extract: one gram of the enriched extract obtained by the process, dissolved in 100 ml of ultrapure water at room temperature and under continuous stirring, has a pH comprised from 5.5 to 7.5, preferably comprised from 6 to 7, for example equal to 6.5 (if the pH under the same conditions had different values, for example if the pH were higher than 4 or lower than 11, the polysaccharides identified as HA could undergo degradation).

The process of the present invention, a process carried out according to techniques and equipment known to the person skilled in the art in the field of extractions, provides (i) for a step of identifying and selecting one or more natural fungi as a starting plant material of a glycosaminoglycan (HA). Said starting plant material may be represented by one or more natural fungi in dry and/or dried form. Preferably, said starting plant material may comprise or, alternatively, consist of at least one fungus belonging to the subkingdom *Dikarya,* preferably the division *Basidiomycota;* more preferably the species *Tremella fuciformis Berk.*

Preferably, in step (i) said one or more natural fungi are identified and selected based on their maturation and growth.

The process of the present invention provides, in addition to (i), also optionally (ii) for a step of carrying out a crushing or pulverisation of the starting plant material to obtain a starting plant material in a certain form such as, for example, flakes or granules or powder and a certain size.

Preferably, the starting plant material which is subjected to the subsequent extraction step in step (iii) could be whole (i.e., a single piece, e.g. a whole fungus), or it could already be a crushed (chopped or flaked) or pulverized (into granules, powder or pellets) plant material in optional step (ii), if desired.

Preferably, said whole fungus may, for example, be a fresh or dry or dehydrated fungus. For example, said freshly harvested whole fungus can be composed of more than 80% water. For example, the percentage of water may range from 85 to 95% of the fresh weight.

Preferably, following step (ii), said starting plant material is reduced in size if it is started, for example, from a whole fungus or from small pieces or flakes of fungus, by using, for example, known mechanical crushing or pulverisation means until reaching an average size comprised from about 5 mesh to about 100 mesh; preferably comprised from 10 mesh to 80 mesh; more preferably comprised from 15 mesh to 60 mesh; more preferably still comprised from 20 mesh to 40 mesh, for example equal to 20 mesh.

In case the starting plant material is crushed or pulverized (according to a technique and equipment known to the person skilled in the art) in step (ii), preferably said starting plant material may have, for example, an average particle size distribution of said starting plant material preferably comprised from 500 µm to 2,500 µm, more preferably comprised from 800 µm to 1,800 µm, even more preferably comprised from 900 µm to 1,200 µm. For example, 20 U.S. mesh are 841 microns or 0.841 millimetres.

Preferably, the starting plant material provided in step (iii) (from step (ii)) is a plant material crushed into pieces or flakes, or pulverized or into granules or pellets.

Preferably, the starting plant material provided in step (iii) (from step (ii)) is preferably a dry or dried material, i.e., it is a starting plant material containing, for example, an amount by weight of water comprised from about 2% to 20%, preferably comprised from 5% to 15%, even more preferably comprised from 8% to 10%, with respect to the total weight of the starting plant material.

The process of the present invention provides, in addition to (i) and optionally (ii), also (iii) a step of extracting said glycosaminoglycan (HA) from the starting plant material obtained from step (ii) with an extraction solvent.

As mentioned, the natural fungus, or the plurality thereof, identified and selected in step (i) may be crushed or pulverized in the optional step (ii). Subsequently, the natural fungus, or the plurality thereof, crushed or pulverized in the optional step (ii) is subjected to at least one step (iii) of extracting said glycosaminoglycan (HA) from the starting plant material obtained from the optional step (ii) with an extraction solvent; preferably, an extraction solvent comprising water; even more preferably, with an extraction solvent consisting of water (for example, distilled or bi-distilled water) to give an extract of said glycosaminoglycan (HA) in said extraction solvent, preferably water. Schematically it can be said that, at the beginning of step (iii), there is the starting plant material in the extraction solvent, for example water, all under stirring to give a sort of maceration of the starting plant material; whereas, at the end of step (iii) it happens that said HA is extracted, as a polysaccharide together with other polysaccharides not of interest, from said starting plant material, thanks to the extraction solvent in which there will also be a part of the starting plant material.

Preferably, in step (iii) said extraction solvent may be selected from an aqueous extraction solvent and only water; more preferably, said extraction solvent may be only water, e.g. distilled or bi-distilled water, to give a solution extract of said glycosaminoglycan (HA).

The aqueous extraction solvent (or aqueous solution) is preferably a hydroalcoholic mixture in which the alcohol, preferably a C2-C6 aliphatic alcohol such as, for example, ethanol is present in a percentage by weight comprised from 0.1% to 50%, more preferably comprised from 0.5% to 25%, even more preferably comprised from 1% to 15%, with respect to the total weight of the extraction solvent, for example in a percentage by weight of about 1.5%, 2%, 2.5%, 3%, 3.5%, 4%, 4.5%, 5% 7.5%, 10%, 20%.

The water is preferably distilled or bi-distilled water.

Preferably, said hydroalcoholic mixture has a water:alcohol weight ratio comprised from 1:5 to 5:1, preferably comprised from 1:4 to 4:1, more preferably comprised from 1:3 to 3:1, more preferably still comprised from 1:2 to 2:1, for example 1:1.

In step (iii), the starting plant material is loaded continuously or in batch into an extraction container or apparatus equipped for example with mechanical stirring means, heating means, filtering means and temperature and pressure control means.

In step (iii), the starting plant material is then subjected to an extraction, in said extraction container or apparatus, by means of the extraction solvent, so that the hyaluronic acid or salt thereof passes into solution in the liquid phase. The liquid phase will also contain other polysaccharides.

In the event that, during the extraction step (iii), for example, with water, there is a part of the starting plant material in solid form that prevents or hinders extraction, a physical separation of the solid part from the extraction solvent may optionally be provided, so as to allow a good extraction yield.

The extraction of step (iii) is conducted using a ratio of [weight of starting plant material]: [volume of the extraction solvent] comprised from 1:1 to 1:90, preferably comprised from 1:10 to 1:90, more preferably comprised from 1:20 to 1:75, even more preferably comprised from 1:40 to 1:60, for example 1:3, 1:5, 1:15, 1:25, 1:45, 1:50 or 1:55. The extraction of step (iii) is conducted in a time comprised from 1 minute to 12 hours, preferably from 10 minutes to 9 hours, even more preferably from 15 minutes to 4 hours, for example during about 30 minutes, 45 minutes, 60 minutes, 90 minutes, 120 minutes, 150 minutes, or 180 minutes.

Step (iii) is preferably conducted at atmospheric pressure (P=1 Atm. at 20-25 °C), and at an extraction solvent temperature comprised from 10 °C to 90 °C, preferably comprised from 20 °C to 60 °C, even more preferably from 35 °C to 55 °C, for example of about 25 °C, 30 °C, 40 °C, 44 °C, 48 °C, or 50 °C.

Step (iii) may also preferably be conducted at an extraction solvent temperature comprised from 95 °C to 100 °C. Preferably, a pH value of the extraction solvent in step (iii) is comprised from 3 to 10, preferably from 3.5 to 9, more preferably from 4 to 8, even more preferably comprised from 5 to 7, for example at a pH value of about 4.5; 5.5; 6; 6.5; 7.5; 8.5; or 9.5.

Preferably, step (iii) is carried out twice.

The process of the present invention provides, in addition to (i), optional (ii), and (iii), also (iv) at least one step of enzymatic hydrolysis.

Preferably, a proteolytic enzyme or a mixture of proteolytic enzymes is employed in step (iv).

Preferably, in step (iv) a mixture of enzymes chosen from the group comprising or, alternatively, consisting of amylase, cellulase (preferably endoglucanase), bromelain and pectinase is employed.

Preferably, in step (iv) a mixture of enzymes comprising at least one enzyme chosen from the group comprising or, alternatively, consisting of amylase, cellulase, bromelain and pectinase is employed.

Preferably, in step (iv) a mixture of enzymes comprising at least two enzymes chosen from the group comprising or, alternatively, consisting of amylase, cellulase, bromelain and pectinase is employed.

Preferably, in step (iv) a mixture of enzymes comprising or consisting of three enzymes chosen from the group comprising or, alternatively, consisting of amylase, cellulase, bromelain and pectinase is employed.

Preferably, in step (iv) a mixture of enzymes comprising or consisting of three enzymes chosen from the group comprising or, alternatively, consisting of amylase, bromelain and pectinase is employed.

Preferably, the mixture of the three enzymes is employed in a weight ratio of the type 1:1:1 (all three enzymes are in equal parts by weight), or 1:1:2 (only two of the three enzymes are in equal parts by weight with respect to each other, while the remaining enzyme is present in a double amount), or 1:1:3 (only two of the three enzymes are in equal parts by weight with respect to each other, while the remaining enzyme is present in a triple amount), or 1:2:3 (the three enzymes are present in a different ratio with respect to each other). The particular mixture of enzymes chosen from amylase, cellulase, bromelain and pectinase allows to increase the extraction yield and reduce extraction times.

Preferably, said at least one enzymatic hydrolysis step (iv) can be conducted before the extraction step (iii) or after the extraction step (iii) or can be conducted in conjunction with the extraction step (iii). In this latter way, the process of the present invention can provide for a single extraction step (iii), with a good process yield, without having to repeat a further extraction step (iii).

Preferably, in the event that said at least one enzymatic hydrolysis step (iv) is conducted in conjunction with said extraction step (iii) (as if it were a single combined step), the mixture of enzymes is added during the extraction step (iii); preferably, after the optional step of physical separation of the solid part from the extraction solvent. In this case, due consideration should be given to the temperature and pH conditions of step (iii) so as not to inactivate or degrade the enzymes used. The mixture of enzymes used can be dissolved in a hydrolysis solvent, for example water, before being added in conjunction with the execution of step (iii), or after conducting step (iii).

Preferably, the enzymatic hydrolysis step (iv) is conducted in water at a temperature comprised from 15 °C to 80 °C, preferably comprised from 20 °C to 70 °C, more preferably comprised from 25 °C to 60 °C, more preferably still comprised from 30 °C to 50 °C (e.g. about 50 °C), for a time comprised from 0.5 hours to 12 hours, preferably from 1 hour to 10 hours, even more preferably from 2 hours to 8 hours (e.g. about 3-10 hours), to give a solution containing HA extracted from said starting plant material and hydrolysed by enzymatic hydrolysis to be subjected to the concentration step (v).

Preferably, the enzymatic hydrolysis step (iv), conducted before or after step (iii), or in conjunction with step (iii), is carried out by using a volume of water comprised from 10 to 100 times the weight of the starting plant material, preferably comprised from 20 to 80 times, more preferably comprised from 30 to 60 times. Preferably, the mixture of enzymes comprising amylase, cellulase (endoglucanase) and pectinase is added, for example in a 1:1:1 ratio, at a temperature comprised from 15 °C to 80 °C, preferably comprised from 20 °C to 70 °C, more preferably comprised from 25 °C to 60 °C, more preferably still comprised from 30 °C to 50 °C (e.g. about 50 °C ± 2 °C), for a time comprised from 0.5 hours to 12 hours, preferably from 1 hour to 10 hours, even more preferably from 2 hours to 8 hours (e.g. about 3-10 hours), to give a solution containing HA extracted from said starting plant material and hydrolysed by enzymatic hydrolysis to be subjected to the concentration step (v).

Preferably, the enzymatic hydrolysis step (iv) is carried out at a pH value of the solution comprised from 2 to 9, preferably from 3 to 5 or from 5 to 8, more preferably from 3 to 4 (for example 3.5) or from 6 to 7; and/or in said enzymatic hydrolysis step (iv) an amount of enzyme (aqueous solution of the enzyme at 1%-20%, or 2%-10%, or 3%-6% weight/weight or weight/volume) is used in a percentage by volume comprised from 0.001% to 1%, preferably from 0.005% to 0.1%, more preferably from 0.008% to 0.05% (for example 0.01%), with respect to the mass by weight of the starting plant material to be extracted (volume/mass) or, alternatively, with respect to the volume of the solution of the starting plant material to be extracted in the extraction and/or hydrolysis solvent (volume/volume).

Preferably, the pectinase enzyme works and performs its enzymatic activity at a pH value of about 4.9 at 25 °C. Preferably, the cellulase enzyme is an endoglucanase and exerts its enzymatic activity at a pH value of about 5.2 at 25 °C.

Preferably, at the end of the hydrolysis step (iv), a step of inactivating the enzymes used can be provided, in an entirely optional manner, before subjecting said solution containing HA extracted, together with other polysaccharides, from said starting plant material and hydrolysed by enzymatic hydrolysis to the concentration step (v). Inactivation is conducted according to techniques known to the person skilled in the art and may involve, for example, the use of heat, or certain specific inactivation compounds.

Preferably, from the steps (iii) of extraction and (iv) of enzymatic hydrolysis carried out concomitantly, or one after the other or one before the other, a compound is obtained having an average size preferably comprised from 100 mesh to 600 mesh, more preferably comprised from 150 mesh to 400 mesh, even more preferably comprised from 175 mesh to 250 mesh, for example equal to 200 mesh.

The process of the present invention provides, in addition to (i), optional (ii), (iii) and (iv) also (v) at least one concentration step; preferably, at least one vacuum concentration step.

Preferably, the concentration step (v) may be repeated several times, for example 2-3 times, in order to eliminate the water solvent from the HA extract. For example, the concentration step (v) can be conducted using equipment and methods known to the person skilled in the art which can also involve, for example, the use of vacuum, so as to work at a temperature lower than that used in the hydrolysis step (iv) and which takes due account of the extracted HA to avoid deterioration phenomena.

Preferably, the concentration step (v) when conducted under vacuum can be conducted, for example, at a temperature comprised from 40 °C to 70 °C, preferably from 50 °C to 60 °C, more preferably at about 45 °C, and a pressure of less than 1 atmosphere, for example, at a pressure comprised from 0.2 to 0.9 atmosphere, preferably from 0.4 to 0.6 atmosphere, for example 0.5 atmosphere.

Preferably, the concentration step (v) reduces the initial volume of the HA-containing solution by an amount comprised from 20% to 80%, preferably comprised from 40% to 60% of the volume, with respect to the initial volume. For example, at the end of the concentration step (v) the volume of the solution containing the HA is reduced by 50% by volume, compared to the initial volume and, therefore, the solution containing the HA that is obtained at the end of the concentration is twice as concentrated, compared to the initial solution. Thus, an aqueous solution comprising concentrated HA is obtained from concentration step (v).

Preferably, at the end of the concentration step (v), if solid parts are present in the aqueous solution comprising concentrated HA, at least one separation step, for example a physical separation of filtration or centrifugation, can optionally be provided, so as to eliminate the presence of said solid parts.

The process of the present invention provides, in addition to (i), optional (ii), (iii), (iv) and (v), also (vi) at least one HA precipitation step; preferably, at least one precipitation step conducted by using a precipitation solution comprising or, alternatively, consisting of at least one alcohol, for example an aliphatic alcohol, and/or water (precipitation solution).

The present process does not use ultrasound to assist precipitation with or without ethanol.

In the present process, it was chosen to dilute as much as possible the alcohol, for example ethanol, used in the precipitation step, to obtain a sufficient concentration to precipitate the extract without altering its structure and content, also avoiding dirtying the HA obtained. In addition, the ethanol was gradually diluted with water until it was completely removed by recovery thereof, indicating an innovation in the present extraction process with respect to what is already known. Then, the added ethanol is recovered (Figure 1A, (b)) and/or other water is added (Figure 1A, (c)) so as to allow dilution of the concentration of alcohol used, e.g. ethanol, and redissolution until removal of the ethanol (vii).

Preferably, step (vi) of precipitation of the HA is conducted on the concentrated aqueous solution comprising HA, obtained directly from step (v) or following the optional separation step, so as to achieve precipitation of the HA

Preferably, the concentrated aqueous solution comprising HA obtained directly from step (v), is added with the precipitation solution water and/or alcohol, for example ethyl alcohol, and then subjected to the subsequent HA precipitation step (vi).

Preferably, the HA precipitation step (vi) is conducted using a solution comprising water and ethanol, for example 80% ethanol, in a volume ratio comprised from 1:5 to 5:1, preferably comprised from 1:4 to 4:1, more preferably comprised from 1:3 to 3:1, more preferably comprised from 1:2 to 2:1, for example 1:1 or 3:4 (volume/volume). Preferably, the HA precipitation step (vi) is conducted for a time comprised from 1 to 6 hours, preferably comprised from 2 to 4 hours, for example 3 hours or 4 hours, and at a temperature comprised from 50 °C to 85 °C, preferably comprised from 70 °C to 80 °C and pressure 1 atmosphere.

Preferably, the HA precipitation step (vi) is conducted using a solution comprising water and ethanol, for example 80% ethanol, in a volume ratio of 3:4 (v/v, i.e., a volume of ethanol equal to three times the volume of the product of the previous step (v) is added, for 4 hours at a temperature comprised from 50 °C to 85 °C, preferably comprised from 70 °C to 80 °C, and pressure 1 atmosphere.

Preferably, the process of the present invention may provide, in addition to steps (i), optional steps (ii), (iii), (iv), (v) and (vi) also at least one separation step.

Preferably, said at least one separation step may, for example, be a physical separation step of filtration or centrifugation, so as to obtain the HA and the precipitation solution comprising the alcohol used, for example ethanol and water. Preferably, the separation step comprises performing a filtration physical separation step twice in succession.

Subsequently, the process of the present invention provides for a heat treatment step, preferably a pasteurization heat treatment step and a subsequent step of eliminating water (viii), for example, by drying to give dried HA or lyophilization to give lyophilized HA.

The optionally dried or lyophilized HA may for example be subjected to an optional sieving step to obtain a HA as a homogeneous powder of a certain mesh grade, e.g. 200 mesh.

Preferably, the process of the present invention provides for a step of removing the alcohol used (for example ethanol and water) in the precipitation step performed after said precipitation step and before the heat treatment step.

The removal step is carried out by diluting the precipitation solution with water at a temperature comprised from 1 °C to 70 °C, preferably comprised from 5 °C to 60 °C, more preferably comprised from 10 °C to 50 °C, even more preferably comprised from 12 °C to 30 °C, for example equal to 15 °C (at a pressure of 1 atmosphere). Preferably, the water is added to the precipitation solution in a gradual manner, until the ethanol present in the precipitation solution is completely removed.

The Applicant has found that thanks to the process of the present invention, which provides for an extraction step (iii), an enzymatic hydrolysis step (iv), a concentration step (v) and a precipitation step (vi), a HA is obtained having a weight average molecular weight comprised from 1000 kDa to 6000 kDa, preferably comprised from 1500 kDa to 5000 kDa, even more preferably comprised from 2000 kDa to 4000 kDa, for example a weight average molecular weight of about 2500 kDa, or 3000 kDa, or 3500 kDa, or 4500 kDa, or 5500 kDa.

The Applicant has found that at the end of the process of the present invention a product is obtained that contains about 90% by weight of polysaccharides of *Tremella fuciformis* and of these over 85% is represented by HA and/or a salt thereof having a weight average molecular weight comprised from 1500 kDa to 4000 kDa, as reported in the experimental part.

### EXAMPLE 1: Process for preparing said mixture, and said composition comprising said mixture, which comprises or, alternatively, consists of hyaluronic acid or a salt thereof (a salt of the hyaluronate anion) of plant origin.

The process, subject-matter comprises, the following steps:
(i) a step of identifying and selecting one or more natural fungi, fresh or in dry or dried form, belonging to the subkingdom *Dikarya,* the division *Basidiomycota,* and the species *Tremella fuciformis;*
(ii) optionally, a step of carrying out a crushing or pulverisation of the starting plant material;
(iii) at least one step of extracting said hyaluronic acid or salt thereof from the starting plant material obtained from step (i) or (ii) by using water (for example distilled or bi-distilled water), at atmospheric pressure (P=1 Atm. at 20-25 °C), at a temperature of 95 °C and for a time comprised from 15 minutes to 4 hours, to give an aqueous extract of said hyaluronic acid or a salt thereof;
(iv) at least one step of enzymatic hydrolysis of said aqueous extract to give a hydrolysed aqueous extract carried out by means of a mixture of the following enzymes: amylase, bromelain, pectinase with a weight ratio of the type 1:1:1 at 50 °C, for about 3 hours;
   - optionally, a step of inactivating the enzymes used in said hydrolysed aqueous extract;
(v) at least one vacuum concentration step, conducted at about 45 °C, and a pressure of less than 1 atmosphere, of said hydrolysed aqueous extract to give a concentrated aqueous extract containing hyaluronic acid or salt thereof reduced by 50% by volume;
(vi) at least one step of precipitation of said concentrated aqueous extract conducted by using a precipitation solution comprising or, alternatively, consisting of water and ethanol 80%, in a volume ratio 3:4 (volume/volume) at a temperature comprised from 50 °C to 85 °C and pressure of 1 atmosphere for 4 hours to give a precipitated aqueous extract;
(vii) at least one step of removing, from said precipitated aqueous extract, said precipitation solution to give an intermediate aqueous extract; step (vii) being carried out by diluting with water the precipitation solution obtained from step (vi) at a temperature comprised from 10 °C to 50 °C, at a pressure of 1 atmosphere, until the ethanol present in the precipitation solution is completely removed;
(vii-1) at least one step of pasteurizing said intermediate aqueous extract to give a thermally treated aqueous extract.
(viii) a step of eliminating the water by dehydration or lyophilization, to give a dry extract;
   - optionally, a step of sieving, for example at 200 mesh, and packaging the dry extract.

Preferably, the precipitation step provides:
- a sub-step of removing part of the alcohol, preferably ethanol, contained in the precipitation solution (ethanol recovery);
   and/or
- a sub-step of diluting the concentration of ethanol contained in the precipitation solution by adding water to the concentrated aqueous extract;

in order to allow the redissolution of hyaluronic acid or salt thereof.

Preferably, the precipitation step provides for at least one filtration or separation step, possibly preceded and/or followed by an addition of said precipitation solution.

### EXAMPLE 2: HA obtained by the process subject-matter of the present invention

The extract enriched in polysaccharides from T. *fuciformis* comprises:
- total polysaccharides in an amount by weight comprised from 80% to 95% by weight, preferably from 85% to 90% by weight, for example 90.6%, with respect to the total weight of the enriched extract, of which from 75% to 95%, preferably from 80% to 90%, for example 87.8%, of said total polysaccharides are polysaccharides identified as HA having a weight average molecular weight comprised from 1500 kDa to 5000 kDa, preferably comprised from 2000 kDa to 4000 kDa;
- uronic acid in an amount by weight comprised from 1% % to less than 3%, preferably comprised from 1.5% to 2.5%, for example 2.39 by weight, with respect to the total weight of the enriched extract;
- NAG in an amount by weight comprised from 0.01% to less than 0.1%, preferably comprised from 0.04% to 0.08% by weight, with respect to the total weight of the enriched extract;
- water in an amount by weight comprised from 2% to 8%, preferably comprised from 4% to 6% by weight, with respect to the total weight of the enriched extract, for example 7.6% by weight, with respect to the total weight of the enriched extract.

One gram of extract dissolved in 100 ml of ultrapure water at room temperature and under continuous stirring has a pH comprised from 5.5 to 7.5, preferably comprised from 6 to 7, for example equal to 6.5.

For example, table 1 below shows an extract obtained by the process.

**Table 1: characterization of the content of polysaccharides, hyaluronic acid, uronic acid, NAG water and the pH of the extract.**

| **Analysis** | **Value** | **Unit of measurement** |
|---|---|---|
| Total polysaccharides (I.M.) | 90.6 | % (w/w, with respect to the weight of the extract) |
| Total hyaluronic acid (I.M.) | 87.8 | % (w/w, with respect to the weight of total polysaccharides) |
| Uronic acid content (I.M.) | 2.39 | % (w/w, with respect to the weight of the extract) |
| NAG content (I.M.) | < 0.1 | % (w/w, with respect to the weight of the extract) |
| Water content (F.K.W.C.) | 7.6 | % (w/w, with respect to the weight of the extract) |
| pH | 6.5 | 1 gram of extract dissolved in 100 ml of ultrapure water at room temperature and under continuous stirring |

### EXPERIMENTAL PART

### Process for preparing hyaluronic acid from Tremella fuciformis Berk according to the present invention.

### Chemical characterization

### Colorimetric dosage of total content of polysaccharides

6 mg of crude sample (*Tremella fuciformis*) was dissolved in 1 mL of deionized water (carbohydrate solution) and mixed with 1 mL of 5% (W/W) aqueous phenol solution (phenol, Merck Life Science, Rome, Italy) prepared immediately before use in a tube. Subsequently, 5 mL of concentrated sulfuric acid (99%, Merck Life Science, Rome, Italy) was rapidly added to the mixture. After allowing the tubes to stand for 10 minutes, they were stirred for 30 s and placed for 20 minutes in a water bath at room temperature for colour development. Then, light absorption at 490 nm was recorded on a spectrophotometer (Infinite 200 Pro MPlex, Tecan, Männedorf, Switzerland). Reference solutions (6 mg/mL sucrose, Merck Life Science, Rome, Italy) were prepared in an identical manner as above, except that the 2 mL aliquot of carbohydrate was replaced by bi-distilled water. Phenol (Merck Life Science, Rome, Italy) used in this procedure was re-distilled and 5% of phenol in water (W/W) was prepared immediately before measurements. Calibrations were carried out using some standard solutions prepared from the corresponding high purity sucrose sugar. UV was programmed to produce a calibration curve with the following concentrations: 10, 20, 30, 40, 50, 60 and 70 mg/L. The solution was measured in triplicate and expressed as mean (%w/w) ± SD.

### Colorimetric determination of the hyaluronic acid (HA) content

The hyaluronic acid (HA) content of the samples was determined as reported in the literature with some modifications. 6 mg of raw material (*Tremella fuciformis*) were dissolved in 1 mL of deionized water and 10 µL of resuspended samples were moved into new Eppendorf tubes and diluted with 190 µL of deionized water. Then, 1.2 mL of sulfuric acid (Merck Life Science, Rome, Italy) and 0.0125 M of tetraborate (Merck Life Science, Rome, Italy) were moved into the Eppendorf tubes containing the diluted samples, stirred for 20 seconds and then boiled at 100 °C for 5 minutes. After ice-cooling, 20 µL of 0.15% hydroxybiphenyl (Merck Life Science, Rome, Italy) (dissolved in 0.5% NaOH, Merck Life Science, Rome, Italy) were added and stirred; thereafter, 100 µL of each sample was placed in a 96-well plate and the absorbance at 340 nm was measured with a spectrophotometer (Infinite 200 Pro MPlex, Tecan, Männedorf, Switzerland). The data were compared with a calibration curve generated with glucuronic acid (standard curve from 0 to 6000 µg/mL, Merck Life Science, Rome, Italy). The results were expressed as mean (%W/W) ± SD.

### Determination of the water content

The analysis was performed according to the Karl Fischer method. The percentage of water concentration in the sample was determined by KF titration using a Mettler Toledo C20 model Coulometric KF Titrators and an Aquastar CombiCoulomat Fritless Methanol Solution (MilliporeSigma, Burlington, MA, USA) as reagent. The KF titrator was qualified for use prior to analysis by using Hydranal^{®} Water Standard 10.0 (Honeywell Fluka, Mexico City, Mexico).

### Determination of the N-acetyl-glucosamine content

The HPLC system (Agilent, Santa Clara, CA, USA) separated and detected acetylgalactosamine (GalNAc) and N-acetylglucosamine (NAG). The C18 Luna column (Phenomenex, Castel Maggiore, Bologna, Italy) was used to separate GalNAc and NAG at 30 °C in a column furnace. The mobile phase consisted of water and methanol (35:65, Vol/Vol) and was pumped at a flow rate of 0.3 mL/min. The isocratic mode was applied. The injection volume was 20 µL. The reading wavelength was 195 nm. The GlcNAc and NAG working standard solutions were prepared daily from 25 to 200 µg/mL and from 10 to 160 µg/L, respectively, by diluting them in the mobile phase.

### Screening of the metals and sodium content

The metals and sodium were analysed using the United States Pharmacopeia (USP) Method 233. The procedure for determining the metal concentrations aims to detect the elemental impurities, which are generally detectable with inductively coupled plasma mass spectrometry (ICP-MS). A 100 mg sample was weighed into a 50 mL tube, dissolved in 5 mL of ultrapure water and sonicated for 15 minutes. After sonication, the volume was adjusted to 50 mL by adding ultrapure water. The solution was then analysed with the iCAP^{™} PRO XP ICP-OES spectrometer (Thermo Fisher Scientific Inc., Waltham, Massachusetts, USA).

### Experimental protocol for the determination of the molecular weight

The molecular weight of the non-animal hyaluronic acid was determined using a 1% agarose gel, following the method reported in the literature for the determination of HA, with some modifications. Briefly, 0.3 g of agarose (Merck Life Science, Rome, Italy) was dissolved in 30 mL of TAE buffer (48.5 g of Tris base, 11.4 mL of acetic acid and 0.5 M of ethylenediaminetetraacetic acid, EDTA, pH 8; all substances were purchased from Merck Life Science, Rome, Italy). The solution was heated for 30 seconds in a high-powered microwave. The gel was poured into the support and solidified before carrying out a pre-run at 100 V for 45 minutes, using the Mini-Sub Cell GT System (Bio-Rad, Hercules, CA, USA). Meanwhile, the samples were prepared by dissolving 3 mg of crude samples in 1 mL of 1× TAE buffer. Before carrying out the gel, 16 µL of the resuspended sample was placed in a new Eppendorf tube and 4 µL of loading buffer (0.2% Bromophenol Blue, 1 mL TAE 1× and 8.5 mL glycerol, purchased from Merck Life Science, Rome, Italy) and the molecular weights (10 µL Select-HA HiLadder, Echelon Biosciences, Tebu-Bio Srl, Magenta, Italy) were added to each sample. The samples allowed to flow at 100 V until they reached 1 cm from the end of the gel. Then, the gel was hydrated in ultrapure water for 24 hours at room temperature with stirring, and then placed in 30% ethanol with 0.015% Stains All dye (Merck Life Science, Rome, Italy) for 24 hours in the dark. The gel was decolorized for 30 minutes in ultrapure water in the dark prior to image acquisition with the ChemiDoc^{™} Touch Imaging System (Bio-Rad, Hercules, CA, USA). The image obtained was analysed with the Image Lab 3.0 software (Bio-Rad Hercules, CA, USA).

### Particle size distribution

The test product was solubilized in an organic solvent and analysed by HPLC (Agilent, Santa Clara, CA, USA) with a GPC detector (Agilent 1260, Agilent, Santa Clara, CA, USA). The molecular weight evaluation was performed by analysing reference materials at different molecular weights. In particular: 194 Da, 1010 Da, 8500 Da, 19330 Da, 141700 Da, 427500 Da, 1044000 Da, 1250000 Da, and 2500000 Da.

### Determination of pH USP 791

Following the standard protocol, one gram of sample was weighed, dissolved in 100 ml of ultrapure water and stirred continuously at room temperature (25 °C). Once the material was completely dissolved, a pH test was carried out while the sample was continuously stirred. For detection, use was made of the VioLab pH50 pH meter with digital electrode XS 201T DHS and integrated temperature sensor (range: pH: 0.00 - 14.00; mV: 1000; temperature: 0 - 100.0 °C).

### Purity

The purity of the non-animal hyaluronic acid was determined by HPLC analysis (HPLC instrument from Shimadzu, Kyoto, Japan) using the method reported in the literature for the detection of HA. Briefly, 20 µL of TRIS buffer (3.0 g of TRIZMA base, 4.0 g of sodium acetate trihydrate, 1.46 g of sodium chloride, and 50 mg of crystalline bovine serum albumin dissolved in 100 mL of 0.12 M HCl, pH 7.3 with 6 M HCl; all substances were purchased from Merck Life Science, Rome, Italy), 30 µL of AC chondroitinase solution (diluted to 10 U/mL with water), and 20 µL of the test sample solution (200 mg of raw material dissolved in 100 mL of ultrapure water) were pipetted into a 1.5 mL conical vial. The vial was placed in a hot water bath at 37 °C for 3 hours. After cooling to room temperature, the sample was diluted to 1 mL by adding 930 µL of mobile phase A and the mixture was analysed with HPLC-UV and HPLC-HRMS systems. A control solution was prepared by replacing the enzyme aliquot with TRIS buffer.

In particular, for the HPLC-UV method, a Phenomenex Synergi Polar 4 µm 150 × 4.6 mm column was preceded by a Security Guard Polar and maintained at room temperature. The mobile phase A was composed of 340 mg of tetrabutylammonium bisulphate dissolved in 1000 mL of water HPLC grade, while the mobile phase B was composed of 340 mg of tetrabutylammonium bisulphate dissolved in 330 mL of water HPLC grade, then, after the solution is at room temperature, brought to 1000 mL with acetonitrile. An injection volume of 30 µL, a flow rate of 1.1 mL/min and a wavelength of 240 nm were used for the reading. The gradient elution program used was reported in Table 2.

For the HPLC-HRMS method, on the other hand, a Phenomenex Synergi Polar 4 µm 150 × 2.0 mm column was preceded by a Security Guard Polar and maintained at room temperature. The mobile phase A comprised 0.1% of formic acid in ultrapure water, while the mobile phase B comprised 0.1% of formic acid in acetonitrile. An injection volume of 5µL and a flow rate of 0.200 mL/min was used for the reading. The implemented gradient elution program was reported in Table 3.

**Table 2. Gradient elution program.**

| **Time** (min) | Mobile phase B% |
|---|---|
| 0.00 | 20 |
| 7.00 | 65 |
| 12.00 | 65 |
| 12.50 | 20 |
| 22.50 | 20 |

**Table 3. Gradient elution program implemented.**

| **Time** (min) | Mobile phase B% |
|---|---|
| 0.00 | 20 |
| 4.00 | 65 |
| 9.50 | 65 |
| 10.00 | 20 |
| 15 | 20 |

### NMR analysis

The solid-state 13C NMR spectrum with magic angle spinning was acquired on a Bruker Avance III 500 spectrometer (Fällanden, Switzerland) equipped with an 11.75 T wide hole magnet, operating at frequencies of 500.13 MHz for 1H and 125.77 MHz for (13)C. A 4 mm triple resonance probe with magic angle spinning (MAS) was used in all experiments. The samples were packed on a zirconia rotor and made to rotate at a MAS speed of 11-15 kHz. For the cross-polarization MAS experiments (13)C, 55 and 28 kHz RF fields were used for excitation and initial decoupling of protons, respectively. During the CP period, the RF (1)H field was increased by increments of 100, while the RF (13)C fields were maintained at a constant level. The protons were decoupled from the carbons during acquisition by using a phase modulation two-pulse decoupling method. A 62 kHz moderate ramp RF field was used for spin locking, while the carbon RF field was matched to obtain an optimal signal. The relaxation delay between the accumulations was 5 s and the contact time CP of 0.5 ms. All chemical shifts are reported in δ scale and refer externally to tetramethylsilane at 0 ppm.

### Attenuated total reflection (ATR) Analysis, Fourier transform infrared spectroscopy (FTIR)

The analysis was performed with a Bruker Alpha II instrument (Brukere Optics, Rosenheim, Germany) equipped with an ATR accessory with a monolithic diamond crystal and a DTGS detector. The instrument operated in the range 4000-450 cm-1 with a resolution of 4 cm-1. The OPUS software (release 8.7) was used to process the ATR-FTIR spectra.

### Polysaccharide spectrum by matrix-assisted laser desorption/time-of-flight ionization (MALDI-TOF)

To verify the correct enzymatic extraction process, samples coming from different phases were analysed; in particular, 3.0 µL of raw material (10 mg of raw material dissolved in 1 mL of ultrapure water) were mixed with 3.0 µL of matrix solution (20 mg/mL of 2,5-dihydroxybenzoic acid dissolved in 30:70 (v/v) acetonitrile: 0.1% TFA in water, 1mM NaCl; all substances were purchased from Merck Life Science, Rome, Italy). 0.5 µL of each mixture was spotted onto a MTP 384 ground steel target plate (Bruker Daltonics GmbH, Bremen, Germany) in triplicate and allowed to dry at room temperature overnight. MALDI-TOF spectra were acquired with the Ultraflextreme mass spectrometer (Bruker Daltonics GmbH, Bremen, Germany) equipped with a 2 Nd Smart-beam laser: YAG operating at 355 nm, using FlexControl software (version 3.3, Bruker Daltonics GmbH, Bremen, Germany). The samples were analysed in a linear manner and the acquisition of the positive ions was obtained with a mass/charge ratio of 300-5000 m/z. The mass spectra obtained are 6000 shots per each sample; the random walk function was activated with partial sample mode. The analysis was carried out according to the data reported in the literature [Lopez-Garcia, M.; Garcia, M.S.; Vilarino, J.M.; Rodriguez, M.V. MALDI-TOF to compare polysaccharide profiles from commercial health supplements of different mushroom species. Food Chem. 2016, 199, 597-604], [Gitsomboon, S.; Ratanapornsompong, G.; Ongphiphadhanakul, B.; Thongpradit, S.; Chanprasertyothin, S.; Chailurtkit, L.O.; Nimitphong, H. Tremella fuciformis beverage improves glycated hemoglobin A1c and waist circumference in overweight/obese prediabetic subjects: A randomized controlled trial. BMC Nutr. 2024, 10, 44].

### Statistical analysis

The experimental procedures were conducted on three independent analyses, with the resulting data presented as mean values accompanied by the respective standard deviations. The statistical significance of the multiple means was evaluated with the variance analysis methodology by using Prism GraphPad 9.5.0 statistical software. The statistical significance threshold was set at p <0.05.

***In vitro* analysis:** multi-barrier *in vitro* sequential model simulating human physiological compartments, including the gastric, intestinal, hepatic, renal, chondrocyte and keratinocyte environments

### Preparation of the agents

The non-animal HA was prepared directly in *Dulbecco's Modified Eagle's Medium* (DMEM, Merck Life Science, Rome, Italy) without phenol red and supplemented with 0.5% foetal bovine serum (FBS, Merck Life Science, Rome, Italy), 2 mM of L-glutamine (Merck Life Science, Rome, Italy) and 1% of penicillin-streptomycin (Merck Life Science, Rome, Italy) for analysis. To confirm the mechanism of action of the non-animal HA (HA content > 80%, Mw: 1500-4000 kDa), the sodium hyaluronate (HA content > 92%, Mw: 100-1000 kDa; Merck Life Science, Rome, Italy), commonly available commercially, was also analysed as a reference.

For both agents studied, the concentration used was 0.01%, prepared from a 1% stock solution.

### Cell cultures

The GTL-16 cell line, a clonal derivative of a poorly differentiated human gastric cancer, was supplied by the Histology Laboratory of the University of Eastern Piedmont (UPO, Novara, Italy). The cells were maintained in *Advanced Dulbecco's Modified Eagle Medium* (Adv DMEM; ThermoFisher Scientific, Waltham, MA, USA) supplemented with 10% of FBS (Merck Life Science, Rome, Italy), 2 mM of L-glutamine, and 1% of penicillin-streptomycin (Merck Life Science, Rome, Italy). The cultures were incubated at 37 °C in a humidified atmosphere containing 5% of CO₂. For the experiments, 2 ×10⁴ cells were inserted into a 6.5 mm polyethylene terephthalate (PET) translucent Transwell^{®} insert with 0.4 µm pores (Greiner bio-one, Kremsmünster, Austria) in a 24-well plate. Prior to the start of the experiments, the cells were incubated for 8 hours with DMEM without phenol red and FBS (GIBCO^{®}, ThermoFisher Scientific, Waltham, MA, USA) but containing 1% of penicillin-streptomycin, 2 mM of L-glutamine and 1 mM of sodium pyruvate (Merck Life Science, Rome, Italy) to synchronize them. For the experiments, the medium was adjusted to pH 3.

The human colorectal carcinoma cells, Caco-2, were purchased from the *American Type Culture Collection* (ATCC, Manassas, VA, USA) and cultured in Advanced Dulbecco's Modified Eagle's Medium/Nutrient F-12 ham (Adv DMEM/F12, GIBCO^{®} ThermoFisher Scientific, Waltham, MA, USA) containing 10% of FBS, 2 mM of L-glutamine and 1% of penicillin and streptomycin (all produced by Merck Life Science, Rome, Italy) in an incubator at 37 °C and 5% of CO2. The cells used in the experiments were at 26-32 passages to maintain the characteristics of permeability and integrative para-cellular transport. To perform the study, 2 × 10⁴ cells were plated on a 6.5 mm Transwell^{®} with a 0.4 µm polycarbonate membrane insert (Corning Costar, New York, NY, USA) in a 24-well plate. Prior to the start of the experiments, the cells were incubated for 8 hours with DMEM without phenol red and FBS (GIBCO^{®}, ThermoFisher Scientific, Waltham, MA, USA) but containing 1% of penicillin-streptomycin, 2 mM of L-glutamine and 1 mM of sodium pyruvate (Merck Life Science, Rome, Italy) to synchronize them. In addition, prior to stimulation with Transwell^{®}, the culture medium on the apical side was adjusted to pH 6.5, which corresponds to the pH of the lumen of the small intestine. In contrast, the pH 7.4 on the basolateral side was adjusted to mimic blood.

The human hepatocellular carcinoma epithelial-like cells, HepG2, were obtained from ATCC (Manassas, VA, USA) and maintained in Adv DMEM (GIBCO^{®} ThermoFisher Scientific, Waltham, MA, USA), supplemented with 10% of FBS (Merck Life Science, Rome, Italy), 2 mM of L-glutamine (Merck Life Science, Rome, Italy) and 1% of penicillin-streptomycin (Merck Life Science, Rome, Italy). The cells were incubated at 37 °C in a humidified atmosphere containing 5% of CO₂. This cell line is often used for hepatic metabolism studies when the passages are comprised between 90 and 95 and after reaching 80-90% of confluence. For scientific tests, 2 × 10⁵ cells were plated in a Transwell^{®} system. Prior to the start of the analysis, the cells were incubated for 8 hours with DMEM without phenol red and FBS (GIBCO^{®}, ThermoFisher Scientific, Waltham, MA, USA) but containing 1% of penicillin-streptomycin, 2 mM of L-glutamine and 1 mM of sodium pyruvate (Merck Life Science, Rome, Italy) to synchronize them.

The immortalized human juvenile rib chondrocyte line, T/C-28a2 (Merck Life Science, Rome, Italy), was maintained in Adv DMEM medium (GIBCO^{®} ThermoFisher Scientific, Waltham, MA, USA) supplemented with 10% of FBS (Merck Life Science, Rome, Italy), 2 mM of L-glutamine (Merck Life Science, Rome, Italy) and antibiotics (50 U/mL penicillin and 50 µg/mL streptomycin; Merck Life Science, Rome, Italy). The cells were cultured at 37 °C in a humidified atmosphere with 5% of CO₂ and 95% of relative humidity. This cell line is usually used between steps 3 and 10. For the experiments, 1 × 10⁶ cells were plated on a 6-well plate and incubated for 8 hours with DMEM without phenol red and FBS (GIBCO^{®}, ThermoFisher Scientific, Waltham, MA, USA) but containing 1% of penicillin-streptomycin, 2 mM of L-glutamine and 1 mM of sodium pyruvate (Merck Life Science, Rome, Italy) to synchronize them.

The HEKa adult epidermal human keratinocyte line was purchased from ATCC (Manassas, VA, USA) and cultured in Adv DMEM medium (GIBCO^{®}, ThermoFisher Scientific, Waltham, MA, USA) supplemented with 10% of FBS (Merck Life Science, Rome, Italy), 2 mM of L-glutamine (Merck Life Science, Rome, Italy), 2 mM of L-glutamine and 1% of penicillin-streptomycin (Merck Life Science, Rome, Italy) and maintained in an incubator at 5% of CO₂ and 95% humidity. For analysis, 1 × 10⁶ cells were plated on a 6-well plate and incubated for 8 hours with DMEM without phenol red and FBS (GIBCO^{®}, ThermoFisher Scientific, Waltham, MA, USA) but containing 1% of penicillin-streptomycin, 2 mM of L-glutamine and 1 mM of sodium pyruvate (Merck Life Science, Rome, Italy) to synchronize them.

The human embryonic kidney cell line HEK293 was purchased from ATCC (Manassas, VA, USA). The cells were maintained in Adv DMEM (GIBCO^{®}, ThermoFisher Scientific, Waltham, MA, USA) supplemented with 10% of FBS (Merck Life Science, Rome, Italy), 2 mM of L-glutamine (Merck Life Science, Rome, Italy) and 1% of penicillin-streptomycin (Merck Life Science, Rome, Italy). The cells were incubated at 37 °C in a humidified atmosphere containing 5% of CO₂. For the experiments, 1 × 10⁶ cells were plated on a 6-well plate. Prior to the start of the analysis, the cells were incubated for 8 hours with DMEM without phenol red and FBS (GIBCO^{®}, ThermoFisher Scientific, Waltham, MA, USA) but containing 1% of penicillin-streptomycin, 2 mM of L-glutamine and 1 mM of sodium pyruvate (Merck Life Science, Rome, Italy) to synchronize them.

### Experimental protocol

The experiments were organized into five separate and linked steps (Figure 9), corresponding to specific cell types, to explore the metabolism of non-animal HA and of the sodium hyaluronate. The GTL-16 cells were used in the initial step to assess cytotoxicity in a time-dependent study, from 1 to 4 hours. A Transwell^{®} system was used to examine the effects on an *in vitro* gastric barrier. Cell viability, production of radical oxygen species (ROS), barrier integrity, inflammation and intracellular and extracellular HA contents, and the molecular weights in supernatants were analysed.

In the second step, the basolateral environment of the gastric barrier was successfully used to explore the intestinal function from 2h to 6h, using Caco2 cells and analysing cell viability, ROS production, barrier integrity, inflammation and intracellular and extracellular HA contents and the molecular weight of both substances tested in the supernatants.

In the third step, the basolateral environment of the intestinal barrier was used to explore the hepatic environment in HepG2 cells for 24 hours, analysing cell viability, ROS production, barrier integrity, inflammation, and intracellular and extracellular HA contents and the molecular weights in the supernatants.

Finally, in the fourth and final step, basolateral hepatic barrier environments were used to explore the effects on chondrocytes, keratinocytes, and renal cells for 24 hours, using T/C-28a2, HEKa, and HEK293 cells, respectively. Cell viability, ROS production, inflammation and intracellular and extracellular HA contents, and the molecular weight were analysed in each of these compartments. In addition, the hyaluronidase levels were analysed in the intestinal, hepatic and renal compartments.

### In vitro gastric barrier model

The GTL-16 cells were seeded on the apical side of a 24-well Transwell^{®} permeable substrate and the trans-epithelial electrical resistance (TEER) was measured continuously for 7 days using an EVOM3 voltmeter coupled with STX2 wand electrodes (World Precision Instruments, Sarasota, FL, USA). The experiments began when the TERR exceeded 150 Ω-cm2. The culture medium was changed by adding chloric acid (HCl, Merck Life Science, Rome, Italy) to obtain a pH 3 on the apical side for 60 minutes, as reported in the literature [Lourengo, B. N.; Dos Santos, T.; Oliveira, C.; Barrias, C. C.; Granja, P. L. Bioingegneria di un nuovo modello 3D in vitro di mucosa gastrica per studi di permeabilità dello stomaco. Acta Biomater 2018, 82, 68-78. DOI: 10.1016/j.actbio.2018.10.007].

### In vitro intestinal barrier model

An intestinal barrier model was created using Caco-2 cells. The TEER values were determined with an EVOM3 voltmeter, coupled with STX2 wand electrodes (World Precision Instruments, Sarasota, FL, USA), every 2 days for 21 days, until a TEER value ≥ 400 Ω-cm2 was reached before stimulation. On day 21, the culture medium in the apical and basolateral environments was modified to create different pH conditions: pH 6.5 at the apical level to mimic the lumen of the small intestine and pH 7.4 at the basolateral level to mimic human blood. The cells were incubated for 15 minutes at 37 °C and 5% of CO2; subsequently, the TEER values were measured again to verify stabilization.

### In vitro hepatic barrier model with HepG2 cells

To assess the integrity of the hepatic barrier, an *in vitro* hepatic model was developed using the HepG2 cell line. In this configuration, the cells were placed in the upper chamber of Transwell^{®} culture inserts with a pore size of 0.4 µm and grown in a complete growth medium, with regular medium changes to maintain viability and functionality. Using an EVOM3 voltmeter and STX2 wand electrodes (World Precision Instruments, Sarasota, FL, USA), the TEER was measured to determine the appropriate maturation time for 3.5 × 10⁴ HepG2 cells, which reached a threshold of 486 Ω-cm2. The TEER values were monitored after exposure to detect any barrier integrity interruptions.

### Cell viability test

Cell viability was analysed with a classic technique based on the MTT-based In vitro Toxicology Assay Kit (Merck Life Science, Rome, Italy), following the manufacturer's instructions. At the end of stimulation, all cell types used were incubated with the 1% MTT dye for 2 hours in an incubator at 37 °C, 5% of CO₂ and 95% of humidity, then the violet-formazan crystals were dissolved in an equal volume of MTT solubilization solution. Absorbance was analysed by spectrophotometer (Infinite 200 Pro MPlex, Tecan, Männedorf, Switzerland) at 570 nm with correction at 690 nm, and the results were expressed with respect to the control (0% line), which represented the untreated cells, from five separate tests performed in triplicate.

### Analysis of ROS production

Quantification of the superoxide anion release was performed using a standard protocol based on the reduction of cytochrome C in the supernatants of all cell types used [Molinari, C.; Ruga, S.; Farghali, M.; Galla, R.; Fernandez-Godino, R.; Clemente, N.; Uberti, F. Effetti di una nuova combinazione di estratti naturali sulla degenerazione retinica correlata al glaucoma. Foods 2021, 10 (8). DOI: 10.3390/foods10081885]. Absorbance was measured with a spectrophotometer (Infinite 200 Pro MPlex, Tecan, Männedorf, Switzerland) at 550 nm. The O2 rate was expressed as mean ± SD (%) of reduced cytochrome C nanomoles per microgram of protein versus the control (line 0) of five separate tests performed in triplicate.

### Tumour necrosis factor α (TNFα) ELISA Kit

The TNFα ELISA kit (Merck Life Science, Rome, Italy) was used to quantify it in the GTL-16, CaCo-2, HepG2, T/C-28a2, HEKa and Hek293 cell supernatants according to the manufacturer's instructions. Using a plate reader (Infinite 200 ProMPlex, Tecan, Männedorf, Switzerland), the absorbance of the samples was determined at 450 nm. A calibration curve (range: 24.58 pg/mL-6000 pg/mL) was used to obtain the results, which are presented as mean values ± SD (%) vs. the control (line 0) of five separate tests performed in triplicate.

### ELISA CD44 kit

The presence of CD44 in all cell lysates (GTL-16, CaCo-2, HepG2, T/C-28a2, HEKa and Hek293) was measured, after lysing the cell with Ripa Buffer (composed of 50 mM HEPES, 150 mM NaCl, 0.1% SDS, 1% Triton X-100, 1% deoxycholate, 10% glycerol and a cocktail of protease inhibitors in 1:100 dilution), using the ELISA Human CD44 kit (FineTest, Wuhan, China) according to the manufacturer's instructions. Briefly, 100 µL of each sample were added to each well and the plate was incubated at 37 °C for 90 minutes. At the end of the incubation, the material in each well was removed and the wells were washed twice with the wash buffer. 100 µL of biotin-labelled antibody working solution were added to the above wells and the plate was incubated at 37 °C for 60 minutes. At the end of the incubation, the solution in each well was removed and the wells were washed three times with washing buffer. Then, 100 µL of SABC working solution were added to each well and the plate was incubated at 37 °C for 30 minutes. Upon completion, the wells were washed five times and 90 µL of TMB substrate was introduced into each well. After 10-20 minutes, 50 µL of Stop Solution was introduced into each well and the plate was immediately read at 450 nm with a plate reader (Infinite 200 Pro MPlex, Tecan, Männedorf, Switzerland). A standard curve has been drawn that relates the intensity of the colour to the concentration of the standard (range from 0 to 10 ng/mL). The results were presented as mean percent (%) ± SD versus control (line 0) of five independent experiments conducted in triplicate.

### Hyaluronic acid ELISA kit

After stimulation, the GTL-16, Caco-2, HepG2, T/C-28a2, HEKa and Hek293 cells were lysed with 100 µL of cold 1× phosphate buffered saline (PBS), and the total HA was measured in all lysates and supernatants of all cell types according to the instructions of the hyaluronic acid ELISA kit (CloudClone, Houston, TX, USA). A spectrophotometer (Infinite 200 Pro MPlex, Tecan, Männedorf, Switzerland) was used to measure the samples at 450 nm after the addition of 50 µL of Stop Solution. The results are presented as mean values ± SD (%) versus the control (untreated cells) of five separate experiments performed in triplicate. The results were obtained using a calibration curve with a range comprised from 4.94 ng/mL to 400 ng/mL.

### Hyaluronidase (HAase), ELISA KIT

The human hyaluronidase (HAase) ELISA kit (MyBiosource, San Diego, CA, USA) analysed the presence of hyaluronidase (HAase) in Caco-2, HepG2, and HEK293 cell lysates, according to the manufacturer's instructions. All cell types were lysed with 1× PBS, then transferred to a 100 µL strip well and incubated for 90 minutes at 37 °C. The strips were treated with 100 µL of detection solution A for 45 min at 37 °C, then washed with the washing solution and incubated with 100 µL of detection solution B for another 45 min. After incubation, 90 µL of substrate solution were added and incubated for 20 minutes at 37 °C in the dark. Finally, 50 µL of stop solution were added to stop the enzymatic process and the plate was analysed with a spectrophotometer (Infinite 200 Pro MPlex, Tecan, Männedorf, Switzerland) at 450 nm. The concentration is given as pg/mL with respect to a standard curve (from 0 to 200 ng/mL) and the results are expressed as a percentage (mean ± SD) versus the control (line 0) of five independent experiments performed in triplicate.

### Determination of the molecular weight of HA

The molecular weight of the HA was determined using a 1% agarose gel, following the method reported in the literature. In detail, after each stimulation, 0.3 g of agarose (Merck Life Science, Rome, Italy) was dissolved in 30 mL of Tris-acetate-EDTA (TAE) buffer (48.5 g of Tris base, 11.4 mL of acetic acid and 0.5 M EDTA, pH 8, Merck Life Science, Rome, Italy). The solution was heated for 30 s in a high power microwave. The gel was poured into the substrate and allowed to solidify before performing a pre-run at 100 V for 45 minutes, using the Mini-Sub Cell GT System (Bio-Rad, Hercules, CA, USA). Meanwhile, the supernatant samples of GTL-16, Caco-2, HepG2, T/C-28a2, HEKa, and Hek293 were prepared by mixing 10 µL of each sample with 6 µL of 1× TAE buffer. Then, 4 µL of loading buffer (0.2% Bromophenol Blue, 1 mL of TAE 1× and 8.5 mL of glycerol, Merck Life Science, Rome, Italy) were added to each sample and to the molecular weights (mixture of 5 µL of Select-HA HiLadder and 5 µL of Select-HA Mega Ladder, Echelon Biosciences, Tebu-Bio Srl, Magenta, Italy). The gels were allowed to flow at 100 V until the samples reached 1 cm from the end of the gel. Then, the gel was hydrated for 24 hours at room temperature under stirring; thereafter, the gel was placed in 30% ethanol containing 0.015% of Stains All dye (Merck Life Science, Rome, Italy) for 24 hours in the dark. Finally, the gels were decolorized for 30 minutes in H2O in the dark before image acquisition with a ChemiDoc^{™} Touch Imaging System (Bio-Rad, Hercules, CA, USA). The images obtained were analysed with the Image Lab 3.0 software (Bio-Rad Hercules, CA, USA).

### Colorimetric test based on uronic acid for the hyaluronic acid (HA) content

The assay used to quantify the HA concentration was the same as reported in the literature. Briefly, 10 µL of supernatant samples of GTL-16, Caco-2, HepG2, T/C-28a2, HEKa, and Hek293 were diluted with 1.2 mL of sulfuric acid (Merck Life Science, Rome, Italy) with 0.0125 M of tetraborate (Merck Life Science, Rome, Italy), stirred for 20 s, and then boiled at 100 °C for 5 min. Once the samples were allowed to cool in ice, 20 µL of 0.15% hydroxybiphenyl (Merck Life Science, Rome, Italy) (dissolved in 0.5% NaOH, Merck Life Science, Rome, Italy) were added and stirred; 100 µL of each sample was placed in a 96-well plate and absorbance was measured at 340 nm by a spectrophotometer (Infinite 200 Pro MPlex, Tecan, Männedorf, Switzerland). The obtained data were compared with a calibration curve generated using glucuronic acid (0-2 mg/mL; Merck Life Science, Rome, Italy) and the results were expressed as mean (%w/w) ± SD versus the control (line 0).

### Statistical analysis

The data are the mean ± standard deviations (SD) of at least five biological replicates, analysed in triplicate. To emphasize the differences between the groups, the control was set to 0% and the values were normalized with respect to the control by averaging the triplicate and expressing the results as a percentage. GraphPad Prism 10.2.3 (GraphPad Software, La Jolla, CA, USA), one-way ANOVA with Bonferroni post hoc test or Mann-Whitney U test was used for statistical analysis. Significance was defined as p-value < 0.05.

### Results

### Chemical characterization

### Analysis of the content of non-animal hyaluronic acid

Table 4 illustrates the results relative to the concentrations of sugars/polysaccharides, HA, uronic acid, N-acetylglucosamine (NAG) and water present in the non-animal hyaluronic acid derived from *Tremella fuciformis.* What is evident from the data (Table 4) is that the extract obtained from *Tremella fuciformis* is significantly enriched in polysaccharides, since the concentration of these compounds within the extract is considerably high, constituting about 90.6% of the total weight of the extract. This observation implies that, once perfected, the extraction protocol can be a fundamental method for obtaining polysaccharide extracts with exceptionally high yields. After establishing that the extract resulting from the extraction procedure was indeed a polysaccharide extract of *Tremella fuciformis,* it became indispensable to clarify the characteristics of the polysaccharides. The analysis revealed that of the 90.6% polysaccharides present in the extract, about 87.8% were identified as HA, indicating that the polysaccharide extract acquired from *Tremella fuciformis* is remarkably abundant in HA. Subsequently, the content of uronic acid was less than 3% residual, preferably a content of uronic acid comprised from 1% to less than 3%, preferably comprised from 1.5% to 2.5%, for example 2.5%, indicating an advantageous yield of the extraction process. The scientific literature confirms this result. In particular, it was observed that a low temperature drying process (lyophilization) could maintain the uronic acid content (2.24%) at levels comparable to those of the non-dried fungus (2.87%). Similarly, the content of NAG was also evaluated. The reported data demonstrate a minimal presence of NAG in the extract, which was less than 0.1%, preferably a NAG content comprised from 0.01% to less than 0.1%, preferably comprised from 0.04% to 0.08%. Finally, the water content in the extract was determined, which was found to be about 7.6% of the total extract, preferably a water content in the extract comprised from 2% to 8%, preferably comprised from 4% to 6%, and for example 5%, of the total extract.

In conclusion, the results obtained from the analysis of the non-animal hyaluronic acid of *Tremella fuciformis* showed a high concentration of polysaccharides, in particular of HA, which constituted about 87.8% of the total polysaccharides of the extract. These data suggest that the extraction process allows the extraction of hyaluronic acid powder or of similar molecules. Furthermore, the low presence of NAG residues and the relatively low water content indicate a good efficiency of the extraction process, making this extract a promising candidate for industrial applications.

**Table 4.The data relating to the content of total polysaccharides, HA, Uronic, NAG and Water were expressed in % w/w ± SD. For all analyses, three independent experiments were performed in quadruple. I.M. = internal method; F.K.W.C = Karl Fisher Water Content.**

| **Analysis** | **Value** | **Unit of measurement** |
|---|---|---|
| Total polysaccharides (I.M.) | 90.6 | % (w/w) |
| Total hyaluronic acid (I.M.) | 87.8 | % (w/w) |
| Uronic acid content (I.M.) | 2.39 | % (w/w) |
| NAG content (I.M.) | < 0.1 | % (w/w) |
| Water content (F.K.W.C.) | 7.6 | % (w/w) |

### Analysis of the metals and sodium content

Metals such as arsenic, cadmium, cobalt, chromium, copper, lead, mercury, zinc, nickel, tin, selenium, iron, magnesium and manganese can be present in the sediments of fungus crops, consequently affecting their overall composition. Therefore, a rigorous screening for heavy metals was conducted to eliminate the possibility of contamination in the non-animal hyaluronic acid extract of *Tremella fuciformis.* Table 5 exhibits all the analytical data relating to the metals, which reveal the absence of heavy metals (arsenic, cadmium, cobalt, chromium, mercury, lead, copper and zinc) or their presence in negligible quantities, as well as a minimal presence of non-heavy metals (nickel, iron, manganese, selenium, tin), with the sole exception of magnesium, identified in a concentration of 99.8 ppm. In addition, the sodium content was analysed to verify that the extracted substance is not a salt derivative. The results indicated that the sodium levels comprised 1.77% of the extract in solution, thus confirming that the extract obtained is indeed HA and not a salt derivative (e.g. sodium hyaluronate).

**Table 5. Analysis of metals performed on USP 233. For all analyses, 3 independent experiments were performed in quadruple. The indicated measurement uncertainty corresponds to the extended uncertainty with coverage factor k=2 at a probability level p=95%. The laboratory does not correct the analytical results for the recovery factor. The decision-making rules adopted for the expression of conformity (if any) do not consider the associated uncertainty contribution, unless it is expressly required by the customer to take it into account or is specifically required by the relevant legislation or regulation. When preceded by the symbol "<", the result refers to the lower limit of quantification of the applied method.**

| Analysis | Value | Unit of measurement |
|---|---|---|
| Arsenic (USP <233>) | < 0.5 | ppm |
| Cadmium (USP <233>) | < 0.5 | ppm |
| Cobalt (USP <233>) | < 0.5 | ppm |
| Chromium (USP <233>) | 0.88 | ppm |
| Iron (USP <233>) | 9.76 | ppm |
| Magnesium (USP <233>) | 99.8 | ppm |
| Manganese (USP <233>) | 1.16 | ppm |
| Mercury (USP <233>) | < 0.2 | ppm |
| Nickel (USP <233>) | < 0.5 | ppm |
| Lead (USP <233>) | < 0.5 | ppm |
| Copper (USP <233>) | < 0.5 | ppm |
| Selenium (USP <233>) | < 1 | ppm |
| Tin (USP <233>) | < 0.5 | ppm |
| Zinc (USP <233>) | < 0.5 | ppm |
| Sodium (USP <233>) | 1.77 | % (w/w) |

### Determination of the molecular weight

As illustrated in Figure 2A, the extract showed a molecular weight of over 2000 kDa, indicating that it resembles a high molecular weight variant of HA. As illustrated in the sample chromatogram (Figure 2B, chromatogram obtained from the particle size distribution), the results of all evaluations revealed that the particle size distribution was recorded with Mn 5185 kDa and PD 2.364, supporting the data observed by the agarose gel.

### Analysis of purity and pH of the non-animal hyaluronic acid

To understand if the non-animal hyaluronic acid was classifiable as HA in all respects, a purity was performed by HPLC analysis to understand if sulphated polysaccharide forms were also present. The analysis (Figure 3) revealed that the non-animal hyaluronic acid corresponded to the disaccharide ΔDi-HA produced by enzymatic hydrolysis of Tremella extract with chondroitinase AC. The identity of this disaccharide was verified by comparison with the reference standard ΔDi-HA and detection of protonated and sodium-plated positive ions in its mass spectrum. The same analysis also demonstrated the absence of chondroitin monosulfates 4 and 6, eluted at 6.25 and 5.35 minutes, respectively.

pH is a fundamental parameter that can greatly affect a number of functional properties of a molecule, including colour, flavour, and texture. In this study, the pH of the *Tremella fuciformis* extract was measured and evaluated at the time of its dissolution in an aqueous solution. In addition, to maintain structural integrity, the HA must have a pH greater than 4 and less than 11 when dissolved in solution; otherwise, the HA may undergo degradation. Based on the analysis conducted with USP 791 methodology (Table 6), the non-animal hyaluronic acid has a pH value of about 6.5 pH units, which is within the acceptable range for the application (5.5 - 7.5).

**Table 6. pH analysis. The value is a pH unit of three independent experiments conducted in quadruple.**

| Analysis | Value | Unit of measurement |
|---|---|---|
| pH (USP<791>) | 6.5 | pH unit |

### Results of the physical characterization of the non-animal hyaluronic acid

To verify that the non-animal hyaluronic acid had indeed quality after all extraction steps, the following experiments were performed to characterize the structure of this extract: NMR analysis, IR/ATR spectrum and MALDI-TOF analysis.

### NMR analysis of the non-animal hyaluronic acid

Solid-state NMR spectroscopy is a valuable tool for investigating the molecular structure, the composition and functional groups of solid-state polysaccharides. By studying the 13C CPMAS NMR, it is possible to identify important structural elements and 3D arrangements of the polysaccharide chains. In the (13)C CPMAS spectrum (Figure 4) of the non-animal hyaluronic acid, the (13)C peaks appear very broad due to the carbohydrate polymeric chains in a disordered amorphous state. Methyl carbon appeared at about 16.5 ppm, while carbonyl carbon resonance was around 175.5 ppm. Furthermore, the very broad peak observed between δ = 55 - 85 ppm is bound to the different sites (13)C of the disaccharides, which consist of monomeric units of glucuronic acid and N-acetylglucosamine. The C1 and C1' peaks of the disaccharide appeared at about 101 ppm. The polymeric chains of the disaccharides are connected through glycosidic bonds, forming interconnected networks that constitute several randomly ordered three-dimensional arrangements. The large down-field peak detected in the NMR (1)H MAS spectrum (Figure 4) confirmed the extent of the intra- and inter-chain hydrogen bonding responsible for disordered arrangements.

The chemical shifts (13)C and the number of peaks of hyaluronic acid of animal origin are similar to those reported in the literature. The only difference is related to the resolution of 13C NMR peaks. However, the 13C NMR spectra reported in the literature concerned sodium hyaluronate of animal origin, while here we report the hydrated form of the non-animal hyaluronic acid.

### ATR-FTIR spectroscopy

The ATR-FTIR spectrum of the studied extract was measured to validate the structural and compositional data of the non-animal hyaluronic acid. Attenuated Total Reflectance (ATR) is a sampling method widely used in FTIR spectroscopy, which employs total internal reflection to generate an evanescent wave that penetrates the sample surface. This wave allows to acquire detailed data on the molecular composition and structure. ATR's popularity stems from its versatility in analysing solid and liquid samples in the native state, simplifying the analysis of a wide range of substances. The results showed that the spectrum (Figure 5, curve a) is fully compatible with the structure of the hyaluronic acid in hydrated form, with the most significant bands assigned as follows. The wide band in the range 3600-3000 cm-1 (peak at 3295 cm-1), is due to the O-H and N-H stretching modes bound to hydrogen; the signals at 2923 and 2873 cm-1 are assigned to the asymmetric and symmetric stretching modes of the C-H groups in the CH, CH2 and CH3 groups. In the low frequency region, the bands at 1604 and 1412 cm-1 are attributed respectively to the stretching modes vₐₛ and v_{sym} of the COO- groups; the shoulder at 1645 cm-1 is due to the bending mode of water and the amide I band. The signals at 1133, 1048 and 1027 cm-1 are assigned respectively to C-O-C in the glycosidic, C-O (exocyclic) and C-OH groups. For comparison, the ATR-IR spectrum of the standard HA is reported (Figure 5, curve b). In summary, it was observed that, apart from some differences in relative intensities (linked to possible differences in chain length and sample particle size), the same peaks identified in the non-animal hyaluronic acid can be related to what is known in the literature for standard HA [Pan, N.C.; Pereira, H.C.B.; da Silva, M.L.C.; Vasconcelos, A.F.D.; Celligoi, M.A.P.C. Improvement Production of Hyaluronic Acid by Streptococcus zooepidemicus in Sugarcane Molasses. Appl. Biochem. Biotechnol. 2017, 182, 276-293], [Manju, S.; Sreenivasan, K. Conjugation of curcumin onto hyaluronic acid enhances its aqueous solubility and stability. J. Colloid Interface Sci. 2011, 359, 318-325], [Wu, Y. Preparation of low-molecular-weight hyaluronic acid by ozone treatment. Carbohydr. Polym. 2012, 89, 709-712].

### MALDI-TOF

MALDI-TOF mass spectrometry was used to analyse the different steps of preparation of the non-animal hyaluronic extract. Characterization of HA with MALDI mass spectrometry presents some challenges due to its chemical properties. The HA has poor ionization efficiency and optimization of sample preparation can improve characterization. However, the direct analysis of a highly polydisperse mixture of long HA fragments (>10 kDa) is complicated due to the short fragments suppressing the high mass species ions. In our analysis, the final extract was compared with a non-animal hyaluronic acid extract prior to extraction (referred to as YEF) and with an intermediate extract (referred to as YET). The results illustrated in Figure 6 and in Table 7 demonstrate the changes in the peak population. Higher peaks characterize the initial pulverized YEF compared to the intermediate YET. In both samples (YEF and YET) some peaks corresponding to the HA oligomers were detected, in particular 1516.55 and 1515.37 corresponding to 8mer; 2650.58 and 2649.02 corresponding to 14mer. The difference in molecular weight between high-intensity peaks is quantified at 162 Da, corresponding to a single unit of glucan. The minimum difference is quantified at 17 Da, attributed to chlorine adducts or hydroxyl groups. Another way to interpret the MALDI-TOF spectra is to observe that glucans were detected as sodium ion adducts ([Glcn + Na]+) showing a peak-to-peak mass difference of 162 Da, which is consistent with the repetitive unit of the β-(1→ 3) glucan. Similar results have been documented previously; the fruiting body of *Tremella fuciformis* consists of (1→ 2)- and (1→ 4)-mannose and (1→ 3) -bound glucans. Based on the data obtained, a progressive and pronounced decrease in the number of peaks from the YEF raw material to the non-animal hyaluronic acid extract can be noted. In fact, a molecular weight disparity is observed between high-intensity peaks corresponding to one unit of sugar, specifically glucan. This demonstrates the effectiveness of the extraction and purification methodology employed to obtain the non-animal hyaluronic acid, which is more present in the sample preceding digestion (YEF sample) through an enzymatic process, as represented by an intermediate extract (YET sample). Ultimately, the purified non-animal hyaluronic acid significantly decreases the number of units distinguishable as glucan.

### In vitro analysis

### Analysis of the non-animal HA in an in vitro gastric barrier model

The first experiments were conducted using a Transwell^{®} 3D model of the gastric barrier with GTL-16 cells to evaluate the effects of the tested samples at the gastric level under simulated digestion conditions and to analyse their passage through this environment. This preliminary evaluation was essential to verify cell viability, epithelial integrity, oxidative stress and inflammatory levels and to analyse the amount of HA at the intracellular and extracellular level.

As shown in Figure 10A, both non-animal HA and the sodium hyaluronate maintained cell viability, increasing it compared to the control (p<0.05). Both samples induced a time-dependent increase in cell viability, with the non-animal HA exerting a greater effect (33% more than sodium hyaluronate after 3 hours, p<0.05).

After confirming cell viability, further analyses were performed to assess barrier integrity. As shown in Figure 10B, both the non-animal HA and the sodium hyaluronate effectively preserved and improved barrier integrity compared to the control (untreated cells, p<0.05). In particular, the non-animal HA exerted a more pronounced effect than sodium hyaluronate over time, reaching statistical significance (p<0.05). Subsequently, the production of ROS and TNFα was evaluated, since the gastric cellular environment is the primary site of contact and metabolism following oral administration of compounds. Alterations of the oxidative or inflammatory state at this site may affect both the integrity of the gastric mucosa and the subsequent systemic bioavailability of the compound. As shown in Figure 10C, none of the tested compounds increased ROS production compared to normal physiological conditions. In particular, the non-animal HA maintained a low level of ROS during all periods analysed, better than sodium hyaluronate, although the difference was not significant. Similar results were obtained in the analysis of TNFα production (Figure 10D); in fact, both the non-animal HA and the sodium hyaluronate reduced TNFα production compared to the control, but not significantly. Among the two samples analysed, the non-animal HA induced the greatest reduction in TNFα production.

After conducting these safety analyses, the mechanisms of HA internalization were studied by analysing the expression of its main receptor, CD44, and by quantifying the intra- and extracellular content of HA (Figure 10E and 10F). Sodium hyaluronate significantly increased CD44 expression compared to the control and non-animal HA (p<0.05), consistent with the higher percentage of intracellular HA detected after administration thereof. In contrast, the non-animal HA induced only a moderate increase in CD44 levels, in line with the higher fraction of extracellular HA observed after treatment. With regard to HA determination, a specific ELISA kit was used to verify whether the non-animal HA was absorbed and internalized as HA or whether it could stimulate endogenous HA production in gastric cells. As shown in Figure 10F, the data showed that the two forms of HA, non-animal HA and sodium hyaluronate, were not internalized into cells and were not able to stimulate endogenous HA production. Overall, these results suggest that the sodium hyaluronate is absorbed more efficiently in the stomach, while the non-animal HA crosses the *in vitro* gastric barrier more effectively.

In addition, the content and the molecular weight of the HA in the gastric supernatant were determined to confirm the previously observed results. As shown in Table 8 and in Table 9, the non-animal HA had a content of 90.5% before stimulation, while sodium hyaluronate had a content of 65%. After crossing the *in vitro* gastric barrier, the non-animal HA was demonstrated to maintain a higher overall content compared to the sodium hyaluronate (85% of non-animal HA vs 58.7% of sodium hyaluronate, p<0.05), indicating greater resistance to enzymatic degradation and acidic pH of the gastric compartment, with potential bioavailability compared to the sodium hyaluronate in subsequent absorption phases.

**Table 8: % w/w of all HA forms was normalized on standard curves generated with standard glucuronic acid (0 to 2 mg/mL), analysed at 340 nm by spectrophotometry (Infinite 200 Pro MPlex, Tecan). The data are expressed as means ± SD (%) of five independent experiments performed in triplicate.**

| Plasma fraction | Gastric model (%) |
|---|---|
| Greenluronic^{®} (non-animal HA) | 85 ± 1.03 |
| Sodium Hyaluronate | 58.7 ± 1.12 |

With regard to molecular weight (Figure 11), gel analysis confirmed what was observed previously: the non-animal HA did not undergo large changes in content or molecular weight, with a molecular weight greater than 2000 kDa. Overall, these results suggest that the non-animal HA exhibits good stability in the gastric cellular environment. After confirming that the non-animal HA maintains a high overall concentration after crossing the gastric barrier, further analysis was performed at the intestinal level.

### Analysis of the non-animal HA in an in vitro intestinal barrier model

Further experiments were conducted using a 3D *in vitro* intestinal barrier model to assess safety and obtain further information about intestinal absorption. In this context, both the gastric eluate of non-animal HA and the sodium hyaluronate were tested for 2-6 hours to measure cell viability, TEER values and ROS production. Instead, the TNFα production, CD44 levels, and HA concentration were assessed after 6 hours. The data obtained from cell viability analysis (Figure 12A) demonstrated that, even in an *in vitro* intestinal environment, the gastric eluate of non-animal HA and the sodium hyaluronate increase cell viability compared to the control (p<0.05). Both reach a peak of cell viability after 4 hours, with the non-animal HA significantly higher than sodium hyaluronate (approximately 28%, p<0.05).

TEER analysis (Figure 12B) demonstrates that both the gastric eluate of non-animal HA and the sodium hyaluronate maintained epithelial integrity compared to the control (p <0.05). In particular, the gastric eluate of non-animal HA demonstrated a greater effect than the gastric eluate of sodium hyaluronate at all times of stimulation (p<0.05).

Subsequently, the analysis of ROS production revealed that the gastric eluate of non-animal HA and the sodium hyaluronate were able to induce an even more positive effect (Figure 12C); in fact, the gastric eluate of sodium hyaluronate was able to maintain ROS levels lower than or equal to those of control up to 5 hours later, while the gastric eluate of non-animal HA induced a more positive effect by maintaining ROS levels below those of control even after 6 hours of stimulation.

Furthermore, throughout the time span, the gastric eluate of non-animal HA had a significant effect compared to the gastric sodium hyaluronate eluate (p < 0.05). The analysis of TNFα production also confirmed the stronger effect of the gastric eluate of non-animal HA compared to the sodium hyaluronate (p<0.05), although both reduced production compared to the control (Figure 12D, p<0.05).

The analysis of CD44 revealed that, again, the gastric eluate of sodium hyaluronate is more likely to be internalized than the gastric eluate of non-animal HA, as both increase the CD44 levels compared to the control (Figure 12E, p<0.05), and the sodium hyaluronate increases them further compared to the non-animal HA (34%, p<0.05). In addition, the data obtained from quantification at the intracellular and basolateral levels supported the hypothesis of the greater availability of HA by non-animal HA (basolateral environment, Figure 12F); in fact, the gastric eluate of non-animal HA presents a greater amount of HA that crosses the intestinal barrier and reaches the medium like the human plasma compared to the control (p<0.05) and to the gastric eluate of sodium hyaluronate (about 11%, p<0.05).

In addition, the study analysed hyaluronidase, an enzyme that has been demonstrated to degrade the luminal HA and to be absorbed into the *in vitro* intestinal environment. The investigation revealed that both the non-animal HA in the gastric eluate and the sodium hyaluronate maintained baseline hyaluronidase levels (Figure 12G), with no significant increases compared to the control. This result indicates that neither compound underwent degradation at the cellular level in the gut. The stable profile of hyaluronidase suggests that the non-animal HA in the gastric eluate can cross the *in vitro* intestinal barrier without undergoing premature enzymatic hydrolysis.

In support of the biological data obtained, an analysis of the content and the molecular weight in the intestinal supernatant was conducted to ensure the integrity of the tested agents. As shown in Table 10 and in Table 9, the gastric eluate of non-animal HA had an HA content of 85% after crossing the *in vitro* intestinal barrier, while the gastric eluate of sodium hyaluronate had a content of 58.7%. It was demonstrated that the non-animal HA maintains a higher overall content than the sodium hyaluronate (74% of non-animal HA vs 49.2% of sodium hyaluronate, p<0.05).

**Table 10: the % w/w of all HA forms was normalized on standard curves generated using the glucuronic acid standard (from 0 to 2 mg/mL), analysed at 340 nm by spectrophotometry (Infinite 200 Pro MPlex, Tecan).The data are expressed as means ± SD (%) of five independent experiments performed in triplicate.**

| Plasma Fraction | Intestinal Model (%) |
|---|---|
| Greenluronic^{®} (non-animal HA) | 74 ± 0.98 |
| Sodium Hyaluronate | 49.2 ± 1.02 |

With regard to molecular weight (Figure 13), gel analysis confirmed what was observed for the content: the non-animal HA did not undergo large changes in content or molecular weight, with a molecular weight greater than 2000 kDa.

These results suggested that the non-animal HA showed greater structural stability and resistance to enzymatic and acidic degradation throughout the gastrointestinal tract, supporting its potential for a better intestinal persistence and consequent systemic bioavailability compared to the conventional sodium hyaluronate. After confirming that the non-animal HA maintained a high overall concentration after passage through the intestinal barrier, further hepatic analyses were performed using the *in vitro* intestinal eluate.

### Analysis of non-animal HA in an in vitro model of hepatic environment

Since the liver represents the primary site of compound metabolism after intestinal absorption, subsequent analyses were performed using hepatic cells.

As shown in Figures 14A and 14B, both the intestinal eluted non-animal HA and the sodium hyaluronate significantly increased the hepatic cell viability compared to the control (p < 0.05), without affecting the integrity of the hepatic layer. In addition, the intestinal eluate of non-animal HA exerts a slightly greater effect on both parameters analysed. These data confirmed that both compounds were well tolerated by the hepatic cells after crossing the *in vitro* gastric-intestinal barrier.

The evaluation of ROS production (Figure 14C) revealed a significant reduction in oxidative stress in both treatments compared to the control (p < 0.05). In particular, the intestinal eluate of non-animal HA induced a more pronounced reduction than the intestinal eluate of sodium hyaluronate (about 64%, p<0.05), indicating a greater antioxidant capacity at the hepatic level.

The quantification of TNFα (Figure 14D) showed that both compounds greatly reduced the TNFα production compared to the control (approximately 31%, p<0.05). Once again, the intestinal eluate of non-animal HA showed a significantly greater inhibitory effect than the intestinal eluate of sodium hyaluronate (p<0.05), highlighting its superior anti-inflammatory potential.

With regard to the expression of the CD44 receptor (Figure 14E), both the intestinal eluate of non-animal HA and the sodium hyaluronate elevated the CD44 levels compared to the control (p<0.05), and the intestinal eluate of sodium hyaluronate showed significantly greater induction compared to the intestinal eluate of non-animal HA (p<0.05). This scheme suggests that the sodium hyaluronate was more easily absorbed by the cells, while the non-animal HA remained more available in the extracellular environment. In accordance with these results, HA quantification in the intra-cellular and extra-cellular fractions (Figure 14F) confirmed that the intestinal eluate of non-animal HA led to higher extracellular HA concentrations compared to the control (p<0.05) and the intestinal eluate of sodium hyaluronate (approximately 17%, p<0.05). In contrast, the intestinal eluate of sodium hyaluronate showed an increased accumulation of intracellular HA, in line with the increased expression of CD44.

In conclusion, since the liver is a primary site of HA clearance, we analysed the activity of hyaluronidase in the HepG2 cells. Hyaluronidase activity remained stable after both treatments, but showed a slight, non-significant reduction following exposure to the non-animal HA from the intestinal eluate. This trend is consistent with the literature indicating that hepatic hyaluronidases, particularly hyaluronidase 1 (HYAL1) and hyaluronidase 2 (HYAL2), mediate early degradation of HA and that the increased enzymatic activity is correlated to tissue stress and early hepatic damage. The lack of an increase in hyaluronidase activity suggests that neither form of HA triggered hepatocellular damage or accelerated degradation. In contrast, the reduced activity observed with the intestinal eluate of non-animal HA may partly explain its increased extracellular persistence and slower metabolic turnover in the liver (Figure 14G).

Overall, these results indicate that, at the hepatic level, the non-animal HA maintains cell viability and exerts antioxidant and anti-inflammatory effects, while promoting a more efficient release of HA in the extracellular compartment, supporting the hypothesis that it may reach several targets outside the hepatocytes. In contrast, the sodium hyaluronate tends to accumulate at the intracellular level, suggesting different metabolic management within the hepatocytes.

In this compartment, to confirm the observations made in the hepatic environment, the content and the molecular weight of HA in the hepatic supernatant were determined.

As shown in Table 11 and in Table 9, the intestinal eluate of non-animal HA showed an HA content of about 74%, while the intestinal eluate of sodium hyaluronate showed a content of about 49.2%. The intestinal eluate of non-animal HA maintained a significantly higher overall content compared to the sodium hyaluronate (59% vs 35.9%, p<0.05). However, compared to the initial administration, the contents of both forms of HA were reduced, indicating that a substantial portion of the compounds was metabolized in the hepatocytes and then distributed outside the cells.

**Table 11: % w/w of all HA forms normalized on standard curves generated using the glucuronic acid standard (from 0 to 2 mg/mL), analysed at 340 nm by spectrophotometry (Infinite 200 Pro MPlex, Tecan). The data are expressed as means ± SD (%) of five independent experiments performed in triplicate.**

| Plasma/Metabolised Fraction | Hepatic Model (%) |
|---|---|
| Greenluronic^{®} (non-animal HA) | 59 ± 0.82 |
| Sodium Hyaluronate | 35.9 ± 0.94 |

With regard to molecular weight (Figure 15), the gel analysis showed that the molecular weights of both forms of HA remained constant throughout the *in vitro* hepatic barrier (>2000 kDa for the non-animal HA and <500 kDa for the sodium hyaluronate), despite the observed reduction in content.

Overall, these results confirm the superior stability and persistence of the non-animal HA, subject-matter of the present invention, compared to the conventional sodium hyaluronate during the *in vitro* gastrointestinal and hepatic transit models. Maintaining the content and the molecular weight supports the hypothesis that the non-animal HA may exhibit increased systemic bioavailability and a prolonged biological activity, likely due to the increased resistance thereof to enzymatic degradation and more controlled release profile. In contrast, the sodium hyaluronate appears more susceptible to metabolic degradation, potentially limiting the persistence and systemic efficacy thereof. After confirming that the non-animal HA maintains a fairly constant level in the liver, further analyses were performed on renal cells using the hepatic eluate.

### Analysis of non-animal HA in an in vitro model of the kidney after gastrointestinal and hepatic passage

After hepatic metabolism, the renal processes and HA excretion were studied using human renal epithelial cells, HEK293. This model was used to assess cell viability, oxidative and inflammatory responses, CD44 receptor expression, and intracellular and excreted HA.

As shown in Figure 16A, both the hepatic eluate of non-animal HA and the hepatic eluate of sodium hyaluronate significantly increased cell viability compared to the control (p < 0.05), confirming their safety in the renal cells. The hepatic eluate of non-animal HA showed a slightly superior effect, suggesting a favourable interaction with the renal epithelial cells.

The evaluation of ROS production (Figure 16B) demonstrated that both compounds significantly reduced oxidative stress compared to the control conditions (p<0.05), with the hepatic eluate of non-animal HA exerting a more pronounced antioxidant effect (approximately 70% more than the sodium hyaluronate, p<0.05). These results suggest that the eluate of non-animal HA helped maintain redox homeostasis in the renal cells, a key factor in damage prevention.

Also in this case, the analysis of TNFα production (Figure 16C) revealed a significant decrease in its production after treatment with both samples compared to the control (p <0.05). Ance again, the hepatic eluate of non-animal HA showed a stronger anti-inflammatory effect (about 30% more than the sodium hyaluronate, p < 0.05), consistent with its protective activity observed in other cell models.

With regard to CD44 (Figure 16D), both the hepatic eluate of non-animal HA and the sodium hyaluronate showed a stronger anti-inflammatory effect (approximately 30% increase compared to the control, although not significant), confirming receptor engagement and HA internalization. The sodium hyaluronate showed a slightly higher CD44 upregulation, consistent with an increased cellular absorption. The analysis of HA quantification (Figure 16E) provided fundamental indications of the renal cell function. Both compounds increased the intracellular HA compared to the control, although not significantly; however, the hepatic eluate of sodium hyaluronate showed a markedly higher proportion of HA in the extracellular compartment (p < 0.05).

Finally, the activity of hyaluronidase was also quantified (Figure 16F). The hepatic eluate of non-animal HA and the sodium hyaluronate all maintained the baseline hyaluronidase levels, with the non-animal HA showing a modest decrease compared to the control. In the kidney, hyaluronidase-mediated degradation of the interstitial HA is closely related to the water reabsorption dynamics, particularly under stimulation of the antidiuretic hormone (ADH). The lack of enzymatic activation suggests that neither form of HA alters the renal HA turnover or the water regulation pathways.

Overall, these results demonstrate that the non-animal HA maintains renal cell viability and reduces the oxidative and inflammatory stress. The lower levels of extracellular HA observed after treatment with the non-animal HA suggest that HA is more efficiently absorbed while passing across the *in vitro* gastric-intestinal-hepatic barriers. Still in the excretion compartment, to confirm what was observed biologically in the renal cells, the content and the molecular weight of HA in the supernatant of HEK293 cells were determined. As shown in Table 12 and in Table 9, the hepatic eluate of non-animal HA showed an HA content of 59% after intestinal passage. In contrast, the hepatic eluate of sodium hyaluronate showed a content of 35.9%. After renal passage, the non-animal HA showed an overall higher content than the sodium hyaluronate (7% vs 2.5%, p<0.05), although both values were drastically reduced compared to the hepatic compartment.

**Table 12: % w/w of all HA forms normalized on standard curves generated using the glucuronic acid standard (from 0 to 2 mg/mL), analysed at 340 nm by spectrophotometry (Infinite 200 Pro MPlex, Tecan). The data are expressed as means ± SD (%) of five independent experiments performed in triplicate.**

| Metabolised/Excreted Fraction | Renal Model (%) |
|---|---|
| Greenluronic^{®} (non-animal HA) | 7 ± 0.15 |
| Sodium Hyaluronate | 2.5 ± 0.17 |

However, the residual content observed for the non-animal HA suggested that a measurable fraction of the compound remained intact and was excreted as high molecular weight HA, confirming the greater stability thereof during all intracellular passages. The higher renal recovery of the non-animal HA compared to the sodium hyaluronate further supports the hypothesis that this formulation possesses greater resistance to enzymatic degradation.

With regard to the molecular weight at the renal level (Figure 17), it was not possible to determine the molecular weight of any of the HA forms of the hepatic eluate due to the very low HA content in both samples. However, the non-animal HA showed a higher overall content than the sodium hyaluronate (7% vs 2.5%, p < 0.05), indicating a greater persistence.

Overall, these results suggest that the non-animal HA is more resistant to metabolic degradation than the sodium hyaluronate, maintaining a measurable fraction in the renal compartment, which supports its potential for prolonged bioavailability and delivery to other target tissues, as tested using hepatic eluate.

### Effects of the non-animal HA on chondrocytes after gastrointestinal and hepatic passage

Since the exogenous HA has a direct biological effect on the chondrocytes when administered into articular cartilage, several experiments were conducted to explore the effects of the non-animal HA from the hepatic eluate, compared to the sodium hyaluronate from the hepatic eluate, on chondrocyte viability, ROS production, TNFα production, and HA internalization.

As shown in Figure 18A, both the non-animal HA of the hepatic eluate and the sodium hyaluronate significantly increased chondrocyte viability compared to the control (p<0.05). The non-animal HA showed a slightly higher effect than that of the sodium hyaluronate, confirming its good cellular biocompatibility and potential trophic activity on the cartilage cells.

In addition, the analysis of ROS production (Figure 18B) revealed that both compounds exerted antioxidant effects, reducing oxidative stress compared to the control (p<0.05). Once again, from the hepatic eluate, the non-animal HA induced a more pronounced reduction than the sodium hyaluronate (approximately 1.1-fold, p<0.05), indicating a greater ability to maintain redox balance within the chondrocytes. Similarly, TNFα quantification (Figure 18C) demonstrated that both the hepatic eluate of non-animal HA and the sodium hyaluronate significantly reduced TNFα release compared to the control (p<0.05), with the non-animal HA showing a superior anti-inflammatory effect compared to the sodium hyaluronate (approximately 34%, p<0.05). This result suggests that the non-animal HA may help limit inflammatory processes typically involved in *in vitro* cartilage degradation.

With regard to in-vitro HA internalization, the analysis of the CD44 levels (Figure 18D) showed that both samples significantly increased the CD44 levels compared to the control (p<0.05), with the hepatic eluate of non-animal HA showing a greater increase than the sodium hyaluronate (approximately 27%, p<0.05). This result is particularly relevant, as CD44 is the main receptor mediating HA internalization and downstream signalling in the chondrocytes, and an increase in CD44 indicates that HA is properly internalized in the chondrocytes. In accordance with these results, HA quantification (Figure 18E) revealed significantly higher intracellular HA concentration after treatment with non-animal hepatic eluate than the sodium hyaluronate and control (approximately 17% compared to the sodium hyaluronate, p<0.05). The increased internalization of the non-animal HA suggests an improved interaction with the HA receptors of the chondrocytes and a potential increased stimulation of the intracellular pathways involved in matrix synthesis and *in vitro* tissue repair. In fact, this increased internalization is beneficial, as it supports the bioactivity of HA within the cells, perhaps contributing to the restoration of cartilage homeostasis and the maintenance of the integrity of the extracellular matrix.

After the biological analyses at the chondrocyte level, the content and the molecular weight of the non-animal HA and of the sodium hyaluronate in the intracellular fraction of the chondrocytes were determined. From the data reported in Table 13 and in Table 9, the content of the non-animal HA in the hepatic eluate was determined to be about 64%. In contrast, that of the hepatic eluate of sodium hyaluronate was about 40%. This difference may derive from the fact that the non-animal HA appears to induce a greater endogenous production than the sodium hyaluronate.

**Table 13: % w/w of all HA forms normalized on standard curves generated with standard glucuronic acid (from 0 to 2 mg/mL), analysed at 340 nm by spectrophotometry (Infinite 200 Pro MPlex, Tecan). The data are expressed as means ± SD (%) of five independent experiments performed in triplicate.**

| Metabolized/Intracellular Fraction | Joint Model (%) |
|---|---|
| Greenluronic^{®} (non-animal HA) | 64 ± 1.27 |
| Sodium Hyaluronate | 40 ± 1.30 |

To confirm this, the molecular weight analysis was performed. As shown in Figure 19, the hepatic eluate of non-animal HA maintains its high molecular weight, whereas the hepatic eluate of sodium hyaluronate shows only minimal molecular weight variation.

Overall, these results indicate that the non-animal HA not only maintains its structural integrity and its high molecular weight during *in vitro* metabolism, but also appears to stimulate endogenous HA synthesis at the chondrocyte level. This dual effect, the preservation of exogenous HA and the induction of the endogenous production, suggests a more durable and efficient contribution to the joint matrix homeostasis than the conventional sodium hyaluronate.

### Effects of non-animal HA on the keratinocytes after gastrointestinal and hepatic passage

To further explore the potential systemic biological effects of the hepatic eluate of non-animal HA, further analyses were performed using an *in vitro* model of human keratinocytes. This model was used to evaluate cell viability, oxidative and inflammatory responses, CD44 receptor expression and HA internalization, thus providing indications on the potential of the compound in supporting homeostasis and epidermal regeneration.

As shown in Figure 20A, both the hepatic eluate of non-animal HA and the sodium hyaluronate significantly increased keratinocyte viability compared to the control (p<0.05). The non-animal HA showed a slightly superior effect, confirming its good cyto-compatibility and its potential in promoting skin cell renewal. The evaluation of ROS production (Figure 20B) demonstrated that both compounds effectively reduced oxidative stress compared to the control (p<0.05) also in this compartment. As observed above, the hepatic eluate of non-animal HA induced a more pronounced reduction (approximately 67% compared to the sodium hyaluronate p<0.05), suggesting a stronger antioxidant potential and the ability to preserve redox balance in the epidermal environment. Similarly, TNFα quantification (Figure 20C) showed that both the hepatic eluate of non-animal HA and the sodium hyaluronate significantly reduced proinflammatory cytokine production compared to the control (p<0.05). The non-animal HA showed the most effective anti-inflammatory action (approximately 48% compared to the sodium hyaluronate, p<0.05), supporting its protective role against *in vitro* inflammation-mediated skin damage.

As illustrated in Figure 20D, both treatments significantly increased the CD44 levels compared to the control (p<0.05), with the hepatic eluate of non-animal HA showing a greater upregulation compared to the sodium hyaluronate (approximately 24%, p<0.05). Since CD44 is the main receptor mediating HA internalization, its upregulation indicates an active HA-cell interaction. Consistently, the quantification of intracellular HA (Figure 20E) confirmed that the hepatic eluate of non-animal HA led to a greater accumulation of HA within the keratinocytes compared to the control and the sodium hyaluronate (approximately 45% compared to the sodium hyaluronate, p<0.05). This increased HA internalization represents an advantageous result, as it could support intracellular hydration mechanisms and contribute to epidermal renewal and repair.

Similarly to the previous procedure, the content and the molecular weight of both forms of hepatic eluate were also measured on the *in vitro* skin model to ensure that the amount reaching the target sites is well maintained and not degraded. As shown in Table 14 and in Table 9, the data indicate that the intracellular fraction of the *in vitro* skin model contained HA at a content of 22%, compared to 59% following hepatic metabolism. Furthermore, only a negligible amount of sodium hyaluronate reaches the skin cells, with a content of 14% compared to 35.9% after crossing the hepatocytes.

**Table 14: % w/w of all HA forms normalized on standard curves generated with standard glucuronic acid (from 0 to 2 mg/mL), analysed at 340 nm by spectrophotometry (Infinite 200 Pro MPlex, Tecan). The data are expressed as means ± SD (%) of five independent experiments performed in triplicate.**

| Metabolized/Intracellular Fraction | Skin model (%) |
|---|---|
| Greenluronic^{®} (non-animal HA) | 22 ± 0.56 |
| Sodium Hyaluronate | 14 ± 0.68 |

In addition, the molecular weight analysis (Figure 21) confirmed that the hepatic eluate of non-animal HA maintains its high molecular weight (>2000 kDa), while the hepatic eluate of sodium hyaluronate maintains a molecular weight of <500 kDa.

The results of this study indicate that the non-animal HA is present in the *in vitro* skin model in a structurally intact and bioactive form, thus confirming its greater stability compared to the sodium hyaluronate. The presence of HA at high molecular weight in the skin cells also suggests a potential role in supporting tissue hydration and regeneration, thus highlighting the efficacy of the non-animal HA in maintaining its activity in several *in vitro* barrier models.

### Discussion

*Tremella fuciformis,* commonly called snow fungus or silver ear fungus, is an edible medicinal fungus belonging to the order *Tremellales* and the family *Tremellaceae.* It contains various bioactive compounds, including fatty acids, proteins, enzymes, polysaccharides, phenols, flavonoids, fibres and trace elements. The polysaccharides present in the fruiting bodies, spores, mycelia and fermentation liquids are particularly important. Their composition and molecular weight influence their properties, which range from 8 × 10³ to 6 × 10⁶ Da. The composition depends on the method of extraction, with sugars such as fucose, galactose, xylose, mannose, glucose and glucuronic acid. Different extraction techniques are used depending on the desired application of the extract. For example, the hot water extraction of the fruiting body allows obtaining mannose with structures bound to (1→ 2) and (1→ 4), as well as glucans bound to (1→ 3). The mycelium, on the other hand, contains mannose, rhamnose, glucuronic acid and glucose in various ratios.

In addition, the *Tremella fuciformis* polysaccharides include xylose, mannose, and glucuronic acid bound by α-1,3-glycosidic bonds, with galactose, arabinose, and fucose side chains. The extract also has a significant amount of uronic acid (especially glucuronic acid) and hydroxyl groups, which help maintain the stability thereof over time, preserving its physical and chemical properties, such as electrical conductivity and pH. The polysaccharides are classified into five types: acidic polysaccharides, neutral polysaccharides, acidic oligosaccharides, and cell wall polysaccharides. The studies suggest that the viscosity of these polysaccharides plays a fundamental role in their biological activity, influencing their function in the body.

Based on this evidence, the following study aimed to chemically and physically characterize a polysaccharide extract obtained from *Tremella fuciformis* to evaluate whether it was a type of extract that could be related to the hyaluronic acid. The extraction of HA from the solid tissues involves several steps: digestion (protease), boiling (denatured enzyme), centrifugation, chloroform extraction, centrifugation, dialysis, ethanol precipitation, centrifugation, re-dissolution, digestion and boiling for enzyme denaturation. In addition, HA extraction techniques utilize detergents, enzymes, and/or solvents to break down the structure and isolate the polysaccharide complexes from the tissue. These techniques depend on the chemical hydrolysis of the tissue, resulting in the breakdown of the proteoglycan core. The extraction method proposed in this study does not involve a centrifugation step. In contrast, an ethanol removal and pasteurization step was performed after the ethanol and water precipitation step. This approach made it possible to obtain a polysaccharide extract with properties comparable to that of the hyaluronic acid, a result not obtainable with the conventional extraction methodologies. In particular, the *Tremella fuciformis* extract was used as a natural substitute for the hyaluronic acid. However, these properties were observed based on its physicochemical characteristics rather than its HA content, demonstrating superior effects compared to the hyaluronic acid. In the present study, the extraction process allowed to obtain an almost totally pure HA extract from *Tremella fuciformis,* as will be discussed later. Indeed, as regards the chemical composition of the extract under examination, the analysis revealed that the non-animal hyaluronic acid extract derived from *Tremella fuciformis* exhibited a marginally acidic pH (pH = 6.5), a property presumably attributable to the presence of acidic polysaccharides within the extract. This result is analogous to the reported stability values of HA in aqueous solution. In the context of the stability of HA in aqueous solution at pH 7, it was observed that the degradation rate increases exponentially with increasing temperature. In contrast, an acidic pH environment has been demonstrated to cleave the HA molecule, leading to the release of NAG. In the present study, the stability of a non-animal HA molecule in solution at pH 6.5 was analysed, determining the presence of NAG. The data obtained demonstrated a reduced presence of NAG, which significantly affects the stability of the high molecular weight polymer.

In addition, a high amount of polysaccharides was observed in the non-animal hyaluronic acid (about 90.6%), showing a yield value similar to those reported in the literature (about 89.6%) Given the hypothesis that the extract is rich in hyaluronic acid, the content of this substance was determined with a colorimetric technique. This analysis method is consistent with the data obtained from HPLC analysis. In fact, the non-animal hyaluronic acid demonstrated an HA content equal to about 87.76%, a profile comparable to the published reference data (about 95%), as well as the HPLC analysis revealed the presence of HA, which was revealed as the corresponding disaccharide ΔDi-HA generated by the enzymatic hydrolysis of chondroitinase AC of the *Tremella fuciformis* extract. The characterization of this disaccharide was determined through comparative analysis with the reference standard ΔDi-HA, together with the identification of the protonated and sodium cations observed in its mass spectrometric profile.

These data confirm the presence of hyaluronic acid in the extract, suggesting the success of the extraction process. In addition, the NAG analysis also demonstrated the success of the extraction process, being almost non-existent (value lower than 1%). Finally, it is important to mention that the characterization of the non-animal hyaluronic acid content confirmed the presence of hyaluronic acid by evaluating the molecular weight distribution and particle size. This result corroborates the data reported in the existing literature (molecular weight higher than 2000 kDa) where the polysaccharides are assimilated to the hyaluronic acid for its application in the food, pharmaceutical and cosmetic industries.

In addition, the data obtained from the analysis of the screening of metals, sodium and water content supported the previous data on the presence of hyaluronic acid, excluding the salt thereof (sodium hyaluronate); in fact, the water content was about 7.6%, whereas the sodium content was less than 2% (about 1.77%). With regard to the levels of metals, these were found to be below the limit for human use, confirming the safety of the extraction process.

Further tests were performed to confirm the composition of the non-animal hyaluronic acid. In relation to the NMR analysis, the natural abundance of (13)C in non-animal hyaluronic acid revealed that the spectrum was compatible with the structure of HA, as reported in the literature. In addition, the ATR-IR spectrum showed some differences in the relative intensities (correlated to possible differences in chain length and sample particle size). However, the peaks identified in the non-animal hyaluronic acid were similar to those observed in the literature, confirming their identicalness.

In addition, the non-animal hyaluronic acid has a molecular weight greater than 2000 kDa; in this case, the direct analysis by MALDI mass spectrometry is complicated by the presence of short ionization fragments. The MALDI-TOF analysis incorporated *Tremella fuciformis* powder, the extract after the first step of the extraction process and the final non-animal hyaluronic acid to evaluate disparities in the polysaccharides. A detailed observation of the MALDI-TOF spectra revealed discrepancies in the population of the peaks. The YEF (YEF powder) sample has more peaks than the YET (initial step of the extraction process) sample. The visible difference in molecular weight between the high-intensity peaks is 162 Da, corresponding to one glucan unit and consistent with the b-(1 3)-glucan repeat unit. Based on the data obtained, it can be affirmed that there has been a progressive and marked reduction in the number of peaks from the raw material (YEF powder) to the non-animal hyaluronic acid, observable in the molecular weight between the high-intensity peaks corresponding to one unit of sugar, in particular of glucan. This demonstrates the correct extraction and purification procedure to obtain non-animal hyaluronic acid.

In conclusion, this comprehensive chemical and physical characterization of *Tremella fuciformis* significantly enhances our previous investigations into its benefits for joint and skin health. By systematically analysing its chemical and physical attributes, we aim to further corroborate the growing evidence of *Tremella's* effectiveness as a supplement to improve overall well-being. This research makes our understanding of its potential progress, bridging the gap between its biological effects and the practical applications in health maintenance.

HA has long been recognized as a key component of the extracellular matrix involved in tissue hydration, elasticity, and repair. It continues to attract an increasing scientific attention both as a therapeutic biopolymer and as a nutraceutical ingredient. In recent years, interest has broadened towards understanding the systemic effects of the HA following oral administration; however, despite the widespread use of HA in the medical and cosmetic formulations, its metabolic fate, the absorption dynamics and the multi-organ biological effects remain only partially elucidated, particularly for the new HA-based variants of non-animal origin.

Regarding the *in vitro* analyses, the present study provides a complete evaluation of the metabolic fate and biological activity of an HA of non-animal origin extracted from *Tremella Fuciformis,* using a multi-barrier *in vitro* sequential model that mimics the physiological passage from gastric to systemic distribution. This approach allowed us to examine, for the first time, the stability, absorption, hepatic manipulation, and the biological effects of the non-animal HA in different cellular environments representative of the gastrointestinal, hepatic, cartilage, skin, and kidney tissues. The initial challenge for HA is the maintenance of molecular integrity in the gastric and intestinal environments. In the *in vitro* gastric model, the non-animal HA preserved cell viability and epithelial integrity by limiting oxidative and inflammatory stress, indicating strong acid stability. Further analyses show that the non-animal HA maintains a high content of about 85%, with a reduction by about 5% compared to the amount administered.

In contrast, the sodium hyaluronate shows a reduction by about 6.3%. This lower reduction in content could suggest that the non-animal HA has a greater intrinsic resistance to the gastric environment than the sodium hyaluronate. The molecular weight evaluation also revealed the structural strength of the non-animal HA after crossing the *in vitro* gastric barrier. In addition, the non-animal HA was retained less intracellularly and transferred more efficiently across the gastric barrier, which could facilitate the subsequent *in vitro* intestinal absorption thereof.

At the intestinal *in vitro* level, the non-animal HA has shown an even greater ability to preserve barrier integrity and reduce inflammation, consistently with the data present in the literature indicating that HA modulates the junctional proteins of the intestinal epithelium and the regulatory signalling pathways, resulting in attenuation of inflammation and mucosal protection. The data obtained in this environment indicate that the non-animal HA not only remains stable, but it may also contribute to epithelial protection. Furthermore, the lower induction of the CD44 expression observed for the non-animal HA compared to the sodium hyaluronate could reflect a more efficient translocation process rather than a receptor-mediated absorption. This is consistent with the results of a study by Kimura et al. [Kimura, M.; Maeshima, T.; Kubota, T.; Kurihara, H.; Masuda, Y.; Nomura, Y. Absorption of Orally Administered Hyaluronan. J Med Food 2016, 19 (12), 1172-1179. DOI: 10.1089/jmf.2016.37256], which observed that HA is not degraded or digested in the gastrointestinal tract, but is instead absorbed in the large intestine and subsequently distributed in the tissues.

With regard to the content and the molecular weight of the two forms of HA, an approximate reduction by 11% was observed for the non-animal HA and 9.5% for the sodium hyaluronate between the gastric and intestinal compartments. This decrease is presumably attributable to the degradation that occurs during the transit in the intestinal environment. However, the limited and almost identical reduction observed in both formulations suggests that they can cross the gastric phase while maintaining adequate structural stability. In particular, it has been demonstrated that the residual content of non-animal HA has a higher resistance to acid degradation and retains a high molecular weight. These characteristics may facilitate a more efficient intestinal absorption and an increased systemic availability in the later phases of the *in vitro* metabolism.

The data obtained from the gastric and hepatic compartments are consistent with those reported in the literature on oral administration, thus confirming the validity of the models used and the analysis protocol used [Li, Y.; Meng, Q.; Yang, M.; Liu, D.; Hou, X.; Tang, L.; Wang, X.; Lyu, Y.; Chen, X.; Liu, K.; et al. Current trends in drug metabolism and pharmacokinetics. Acta Pharm Sin B 2019, 9 (6), 1113-1144. DOI: 10.1016/j.apsb.2019.10.001]. However, the analysis of the hepatic model showed a reduced intracellular absorption and an increased extracellular persistence of the non-animal HA compared to the sodium hyaluronate, suggesting that a greater portion may transit towards the peripheral tissues. This result is consistent with the preclinical evidence showing that the high molecular weight HA was detected in the connective tissues 24 hours after oral administration in animal models. In this district, the content and the molecular weight of the two forms of HA are reduced by about 15% for the non-animal HA and 13% for the sodium hyaluronate in the passage from the intestinal to the hepatic environment. This reduction can be attributed to the normal hepatic metabolic processes, during which part of the compound is used or transformed. However, the molecular weight stability observed for both forms indicates that the structure of the hyaluronic acid is not significantly altered, suggesting that the hepatic metabolism primarily affects the amount available rather than the integrity of the molecule.

After characterizing the hepatic compartment, further experiments focused on the renal compartment to evaluate excretion mechanisms. Both the non-animal HA and the sodium hyaluronate have preserved cell viability and reduced oxidative and inflammatory stress, confirming their safety at the renal level. However, while the sodium hyaluronate led to higher levels of excreted HA, the non-animal HA showed reduced levels of extracellular HA, indicating more efficient systemic retention. These results are consistent with the hypothesis that the non-animal HA may persist longer in the medium, thus prolonging the biological effects. The content of the two forms of HA was significantly lower than that observed in the hepatic compartment, with reductions by about 52% for the non-animal HA and about 33% for the sodium hyaluronate. This marked decrease suggests that, following hepatic metabolism, the non-animal HA has been predominantly distributed. This hypothesis is further supported by the molecular weight analysis, which revealed that the low concentration of HA in the renal cells prevented an accurate determination of the MW thereof. This data suggests two potential mechanisms: firstly, a partial degradation of the hyaluronic acid during excretion; secondly, an increased retention of the high molecular weight fraction of non-animal HA in the target tissues. After hepatic metabolism and excretion, the resulting compounds can be studied on peripheral targets, such as skin and joint tissues, in other *in vitro* models.

HA is essential for maintaining the integrity of the connective tissues, especially of the joints; in fact, numerous studies demonstrate the potential of HA in the treatment of joint disorders. The main mechanism of efficacy of HA is chondro-protection, supported by other mechanisms, such as anti-inflammatory effects. The analysis of the TNFα production at the chondrocyte level demonstrated that the non-animal HA maintained anti-inflammatory activity after the gastrointestinal and hepatic passage, indicating the positive effects of this non-animal HA. In addition, one of the main molecular mechanisms underlying the chondro-protective action of HA is the bond thereof with CD44. In fact, it strongly increased the CD44 levels in the chondrocytes, demonstrating to be properly internalized in the cells, where it can exert its beneficial effects. It is worth noting that the non-animal HA maintained a high MW, a data of particular importance, as the researches by comparing HA with different MW demonstrated that, with regard to the treatment of the joint disorders, the HA with higher MW had more positive effects and results than the HA with lower MW. In this district, our results suggest that the non-animal HA can modulate inflammation even after absorption, metabolism, and excretion, reducing TNFα production, as reported in the literature. Furthermore, the non-animal HA bound to CD44 on the chondrocytes in order to exert its biological activity after gastrointestinal transit and hepatic metabolism, supporting the idea that the HA-CD44 bond increases the HA absorption and suppresses the pro-inflammatory processes, as reported in the literature. Another aspect that emerged from this study is the behaviour of the hyaluronidase enzymes in simulated physiological compartments. Hyaluronidases are key regulators of HA turnover in tissues such as the liver and kidney, where they mediate the degradation thereof into low molecular weight fragments. Their over-regulation is often linked to stress or tissue damage. In the multi-barrier model, the hyaluronidase activity remained stable in the intestinal, hepatic and renal compartments. It was not increased by either form of HA, indicating that the endogenous pathways of HA degradation were not activated. The non-animal HA was demonstrated to cause a modest decrease in enzyme activity in the hepatic and renal cells, consistent with its increased extracellular stability and reduced intracellular absorption. The unchanged profile of hyaluronidase suggests that the non-animal HA is less susceptible to enzymatic hydrolysis, thus supporting its greater persistence along the route of metabolization and simulated excretion and its potential for increased systemic activity.

In addition, some studies have demonstrated that daily consumption of HA supplements can positively influence skin cells, improving cellular homeostasis. Indeed, for example, the HA-based formulations have demonstrated a remarkable efficacy in the treatment of a wide range of inflammatory skin diseases. The anti-inflammatory activity of HA was also demonstrated in this study, as the non-animal HA reduced TNFα production more efficiently than the sodium hyaluronate. In addition, HA has been demonstrated to dramatically increase keratinocyte proliferation, thereby improving the epidermal thickness. Furthermore, the interaction between HA and CD44 provides effective methods for the treatment of a number of skin conditions associated with ageing. In the present study, the non-animal HA strongly increased the CD44 levels in the keratinocytes, demonstrating that it is properly internalized in the cells while maintaining its high molecular weight, an important factor, since the HA at high MW has already been demonstrated to promote keratinocyte health with a more positive effect.

In conclusion, the analysis of HA content and molecular weight in the chondrocytes and keratinocytes confirmed the greater stability and bioavailability of the non-animal HA than the sodium hyaluronate. In both cell types, the non-animal HA maintained a higher molecular weight and content, suggesting a reduced degradation and a more efficient distribution to the target tissues. Overall, these results suggest that the non-animal HA may improve persistence and biological function in the joint and skin compartments. Overall, these results demonstrate a consistent model in which the non-animal HA exhibits an increased stability and antioxidant and anti-inflammatory properties. They may suggest a greater systemic availability under *in vitro* conditions than the sodium hyaluronate. The ability of the non-animal HA to preserve its molecular integrity in the gastric and hepatic environment, to effectively cross the intestinal barrier, and to reach the target tissues. This study, for the first time, provides evidence of the systemic metabolism of the non-animal HA by using a multi-barrier *in vitro* sequential model that reproduces the physiological progression of the absorption, as evidenced by the greater homology of the data with the literature. This approach allowed to evaluate the stability of the compound, the transfer dynamics and the biological effects in a context similar to the systemic one.

However, some limitations need to be acknowledged, including the inability to fully capture the complexity of the in-vivo processes, such as the enzymatic degradation by the microbiota, the hormonal regulation, and the tissue-specific absorption kinetics. A future integration of this model could better simulate the dynamic fluidic conditions and the communication among organs.

### Conclusions

In conclusion, the non-animal hyaluronic acid extract of *Tremella fuciformis* (Greenluronic^{®}) taken under consideration in this study, subject-matter of the present invention, proved to be a promising source of hyaluronic acid or structurally similar polysaccharides, with a significant potential in the cosmetic and pharmaceutical industry. The preliminary chemical and physical analyses confirmed the presence of bioactive components, including the hyaluronic acid, within the extract. The molecular characteristics, including the molecular weight and composition, were crucial for the functional properties of the extract, suggesting that a careful selection of the extraction method, subject-matter of the present invention, can optimize the benefits thereof.

The present study demonstrates that HA of non-animal origin, a hyaluronic acid of plant origin obtained from *Tremella* Bianca, exhibits an enhanced absorption and antioxidant and anti-inflammatory effects across multiple *in vitro* biological barriers. With respect to the sodium hyaluronate, the non-animal HA demonstrated a higher systemic transfer, a greater extracellular availability, and a greater activity on the target cells, including chondrocytes and keratinocytes. The results of this study suggest that it maintains the *in vitro* biological activity, as demonstrated by the increased CD44 receptor expression and HA internalization.

These results provide new insights into the systemic behaviour of the non-animal HA and support the use thereof as a sustainable and effective alternative for nutraceutical and therapeutic applications targeting joint and skin health.

### Abbreviations

The following abbreviations are used:
- TFPS: *Tremella fuciformis* polysaccharide
- HPLC: high-performance liquid chromatography
- HPLC-UV: high-performance liquid chromatography-ultraviolet
- HPLC-HRMS: high-resolution mass spectrometry
- UV: ultraviolet spectrometry
- IR: infrared spectrometry
- ATR: attenuated total reflectance
- GC: gas chromatography
- SM: mass spectrometry
- NMR: nuclear magnetic resonance
- GalNAc: acetylgalactosamine
- NAG: N-acetylglucosamine
- USP: United States Pharmacopoeia
- HA: hyaluronic acid
- D2O: deuterium oxide
- FTIR: Fourier transform infrared spectroscopy
- MALDI-TOF: matrix-assisted laser desorption/time-of-flight ionization
- Adv DMEM: Advanced Dulbecco's Modified Eagle Medium
- Adv DMEM/F12: Advanced Dulbecco's Modified Eagle's Medium/Nutrient F-12 Ham
- ATCC: American type culture collection
- CD44: Differentiation cluster 44
- DMEM: Dulbecco's modified Eagle's medium
- ECM: Extracellular matrix
- FBS: Foetal bovine serum
- HA: Hyaluronic acid or salt thereof
- HAase: Hyaluronidase
- HCl: Chloric acid
- HYAL1: Hyaluronidase 1
- HYAL2: Hyaluronidase 2
- MW: Molecular weight
- PBS: Phosphate buffered saline
- PET: Translucent polyethylene terephthalate
- ROS: Oxygen radical species
- SD: Standard deviation
- TAE: Tris-acetate-EDTA
- TEER: Trans-epithelial electrical resistance
- TFP: Tremella fuciformis
- TNFα: Tumour necrosis factor-a

**Table 7. List of representative MALDI-TOF spectra peaks in the 300-5,000 m/z region. The abbreviations comprise: YET = non-animal hyaluronic acid extract before extraction; YEF = non-animal hyaluronic acid intermediate extract; non-animal HA = non-animal hyaluronic acid.**

| Non-animal HA | YET | YEF |
|---|---|---|
| m/z | m/z | m/z |
| 312.697 | 381.001 | 381.103 |
| 330.624 | 454.668 | 459.885 |
| 374.738 | 458.837 | 464.996 |
| 462.842 | 464.888 | 478.402 |
| 510.252 | 478.390 | 524.831 |
| 532.431 | 482.196 | 543.104 |
| 543.860 | 495.822 | 559.075 |
| 565.865 | 502.348 | 581.223 |
| 571.893 | 517.972 | 598.114 |
| 580.831 | 525.960 | 614.146 |
| 587.856 | 543.142 | 636.220 |
| 593.898 | 565.258 | 675.538 |
| 598.787 | 582.102 | 687.308 |
| 603.820 | 613.764 | 705.572 |
| 609.718 | 620.098 | 713.488 |
| 719.971 | 668.689 | 751.652 |
| 770.132 | 688.092 | 765.576 |
| 902.166 | 705.114 | 779.085 |
| 924.212 | 778.659 | 785.838 |
| 945.997 | 833.094 | 796.461 |
| 1122.485 | 850.682 | 806.889 |
| | 867.190 | 823.238 |
| | 1029.267 | 849.652 |
| | 1175.314 | 867.906 |
| | 1191.309 | 995.427 |
| | 1337.360 | 1011.832 |
| | 1353.316 | 1030.180 |
| | 1499.363 | 1157.125 |
| | 1515.347 | 1174.062 |
| | 1661.297 | 1192.391 |
| | 1677.292 | 1319.139 |
| | 1823.270 | 1336.227 |
| | 1839.786 | 1354.493 |
| | 1984.827 | 1481.139 |
| | 2001.761 | 1498.332 |
| | 2146.814 | 1516.549 |
| | 2163.715 | 1643.134 |
| | 2309.045 | 1660.513 |
| | 2325.651 | 1678.650 |
| | 2471.000 | 1805.220 |
| | 2487.098 | 1822.475 |
| | 2633.236 | 1840.685 |
| | 2649.024 | 1967.455 |
| | 2794.832 | 1984.535 |
| | 2811.127 | 2002.688 |
| | 2956.619 | 2129.519 |
| | 2972.900 | 2146.446 |
| | 3118.915 | 2164699 |
| | 3135.261 | 2291.634 |
| | 3280.830 | 2308.450 |
| | 3296.715 | 2326.695 |
| | 3442.620 | 2452.915 |
| | 3459.169 | 2470.398 |
| | 3604.503 | 2488.697 |
| | 3620.750 | 2615.662 |
| | 3766.399 | 2632.355 |
| | 3782.406 | 2650.580 |
| | 3928.011 | 2777.754 |
| | 3944.584 | 2794.233 |
| | 4090.139 | 2812.479 |
| | 4105.915 | 2921.118 |
| | 4267.371 | 2939.020 |
| | | 2956.096 |
| | | 2974.430 |
| | | 3101.218 |
| | | 3118.087 |
| | | 3136.289 |
| | | 3262.422 |
| | | 3280.065 |
| | | 3298.096 |
| | | 3424.925 |
| | | 3441.606 |
| | | 3460.027 |
| | | 3586.663 |
| | | 3603.239 |
| | | 3622.009 |
| | | 3765.512 |
| | | 3783.517 |
| | | 3926.940 |
| | | 3945.246 |
| | | 4088.691 |
| | | 4107.305 |
| | | 4251.836 |
| | | 4269.047 |
| | | 4412.032 |
| | | 4430.799 |
| | | 4592.707 |
| | | 4735.711 |
| | | 4753.176 |
| | | 4915.806 |

**Table 8. The data relating to the HA content were expressed in % w/w ± SD (analyses performed every two months for one year).**

| Non-animal HA | May 2023 | August 2023 | November 2023 | February 2024 | May 2024 |
|---|---|---|---|---|---|
| HA content | 92.0% | 91.2% | 91.2% | 88.2% | 87.8% |

**Table 9: Non-animal HA and sodium hyaluronate content in in vitro compartment models. The data set comprises the percentage content of the hyaluronic acid (HA) in non-animal HA and sodium hyaluronate samples after exposure to various biological models, including stomach, intestine, liver, kidneys, joints, and skin. The results are expressed as means (%) ± SD of five independent experiments performed in triplicate.**

| Sample | Administration (%) | Gastric Model (%) | Intestinal Model (%) | Hepatic Model (%) | Renal Model (%) | Joint Model (%) | Skin Model (%) |
|---|---|---|---|---|---|---|---|
| Non-animal HA | 90.5 ± 1.49 | 85 ± 1.03 | 74 ± 0.98 | 59 ± 0.82 | 7 ± 0.15 | 64 ± 1.27 | 22 ± 0.56 |
| Sodium hyaluronate | 65 ± 1.54 | 58.7 ± 1.12 | 49.2 ± 1.02 | 35.9 ± 0.94 | 2.5 ± 0.17 | 40 ± 1.30 | 14 ± 0.68 |

### Experimental protocol for the reduction of the molecular weight of the hyaluronic acid

An SDS solution at different concentrations was used to modulate the molecular weight of the HA and obtain five different HA molecules with different molecular weights. The fragments to be obtained can be the following: 4000000 Da (4000 kDa; dissolve the sample with an 1% SDS solution; 0.01 g of SDS in 1 mL of ultrapure water), 4000000-2600000 Da (of SDS in 1 mL of ultrapure water), 4000-2600 kDa; dissolve the sample with a 3% SDS solution; 0.03 g of SDS in 1 mL of ultrapure water), 800000-1500000 Da (15000-800 kDa; dissolving the sample with a 5% SDS solution; 0.05 g of SDS in 1 mL of ultrapure water) and less than 300000 Da (300 kDa; dissolving the samples with an 8% SDS solution; 0.08 g of SDS in 1 mL of ultrapure water). Five samples were prepared by weighing 1 mg and dissolving it with 1 mL of different SDS solutions (1% SDS, 3% SDS, 5% SDS and 8% SDS); then, after carrying out all weighings, incubate the samples at 37 °C under stirring for 15 minutes to activate the cutting reaction. These samples were compared with 1 mg of Greenluronic^{®} dissolved with 1 mL of ultrapure water to compare the molecular weight, the hyaluronic and polysaccharide content.

### Experimental protocol for the determination of the molecular weight

The molecular weight of the non-animal hyaluronic acid was determined using a 1% agarose gel, following the method reported in the literature for the determination of HA, with some modifications. Briefly, 0.3 g of agarose (Merck Life Science, Rome, Italy) was dissolved in 30 mL of TAE buffer (48.5 g of Tris base, 11.4 mL of acetic acid and 0.5 M of ethylenediaminetetraacetic acid, EDTA, pH 8; all substances were purchased from Merck Life Science, Rome, Italy). The solution was heated for 30 seconds in a high power microwave. The gel was poured into the substrate and solidified before performing a pre-run at 100 V for 45 minutes, using the Mini-Sub Cell GT System (Bio-Rad, Hercules, CA, USA). 16 µL of previously prepared samples were placed in a new Eppendorf tube, and 4 µL of loading buffer (0.2% Bromophenol Blue, 1 mL of TAE 1× and 8.5 mL of glycerol, purchased from Merck Life Science, Rome, Italy) and the molecular weights (10 µL of Select-HA HiLadder, Echelon Biosciences, Tebu-Bio Srl, Magenta, Italy) were added to each sample. The samples allowed to flow at 100 V until they reached 1 cm from the end of the gel. Then, the gel was hydrated in ultrapure water for 24 hours at room temperature under stirring, and then placed in 30% ethanol with 0.015% Stains All dye (Merck Life Science, Rome, Italy) for 24 hours in the dark. The gel was decolorized for 30 minutes in ultrapure water in the dark prior to image acquisition with the Touch Imaging System (Bio-Rad, Hercules, CA, USA). The image obtained was analysed with the Image Lab 3.0 software (Bio-Rad Hercules, CA, USA). ChemiDoc^{™}

### Polysaccharide spectrum by matrix-assisted laser desorption/time-of-flight ionization (MALDI-TOF)

To verify the correct enzymatic extraction process, samples coming from different phases were analysed; in particular, 3.0 µL of raw material (10 mg of raw material dissolved in 1 mL of ultrapure water or SDS solution) were mixed with 3.0 µL of matrix solution (20 mg/mL of 2,5-dihydroxybenzoic acid dissolved in 30:70 (v/v) acetonitrile: 0.1% TFA in water, 1mM NaCl; all substances were purchased from Merck Life Science, Rome, Italy). 0.5 µL of each mixture was spotted onto a MTP 384 ground steel target plate (Bruker Daltonics GmbH, Bremen, Germany) in triplicate and allowed to dry at room temperature overnight. MALDI-TOF spectra were acquired by the Ultraflextreme mass spectrometer (Bruker Daltonics GmbH, Bremen, Germany) equipped with a 2 Nd Smart-beam laser: YAG operating at 355 nm, using FlexControl software (version 3.3, Bruker Daltonics GmbH, Bremen, Germany). The samples were analysed in a linear manner and the acquisition of the positive ions was obtained with a mass/charge ratio of 300-5000 m/z. The mass spectra obtained are 6000 shots per each sample; the random walk function was activated with partial sample mode. This analysis was performed based on the data reported in the literature.

### Colorimetric dosage of total content of polysaccharides

1 mg of crude sample was dissolved in 1 mL of deionized water (carbohydrate solution) and mixed with 1 mL of 5% (W/W) aqueous phenol solution (phenol, Merck Life Science, Rome, Italy) prepared immediately before use in a tube. Subsequently, 5 mL of concentrated sulfuric acid (99%, Merck Life Science, Rome, Italy) was rapidly added to the mixture. After allowing the tubes to stand for 10 minutes, they were stirred for 30 s and placed for 20 minutes in a water bath at room temperature for the colour to develop. Then, light absorption at 490 nm was recorded on a spectrophotometer (Infinite 200 Pro MPlex colour, Tecan, Männedorf, Switzerland). Reference solutions (1 mg/mL sucrose, Merck Life Science, Rome, Italy) were prepared in an identical manner as above, except that the 2 mL aliquot of carbohydrate was replaced by water. Phenol (Merck Life Science, Rome, Italy) used in this procedure was re-distilled and 5% of phenol in water (W/W) was prepared immediately before measurements. Calibrations were carried out using standard solutions prepared from the corresponding high purity sucrose sugar. UV was programmed to produce a calibration curve with the following concentrations: 10, 20, 30, 40, 50, 60 and 70 mg/L. The solution was measured in triplicate and expressed as mean (%w/w) ± SD [bi-distilled].

### Colorimetric determination

The hyaluronic acid (HA) content of the samples was determined as reported in the literature.

1 mg of raw material was dissolved in 1 mL of deionized water, and 10 µL of resuspended samples were placed in new Eppendorf tubes and diluted with 190 µL of deionized water. Then, 1.2 mL of sulfuric acid (Merck Life Science, Rome, Italy) and 0.0125 M of tetraborate (Merck Life Science, Rome, Italy) were moved into the Eppendorf tubes containing the diluted samples, stirred for 20 seconds and then boiled at 100 °C for 5 minutes. After ice-cooling, 20 µL of 0.15% hydroxybiphenyl (Merck Life Science, Rome, Italy) (dissolved in 0.5% NaOH, Merck Life Science, Rome, Italy) were added and stirred; thereafter, 100 µL of each sample was placed in a 96-well plate and the absorbance at 340 nm was measured with a spectrophotometer (Infinite 200 Pro hydroxybiphenyl MPlex, Tecan, Männedorf, Switzerland). The data were compared with a calibration curve generated with glucuronic acid (standard curve from 0 to 1000 µg/mL, Merck Life Science, Rome, Italy). The results were expressed as mean (%W/W) ± SD.

### NMR analysis

A C NMR spectrum with magic angle spinning was acquired on a Bruker Avance III 500 spectrometer (solid state, Fällanden, Switzerland) equipped with an 11.75 T wide bore magnet, operating at frequencies of 500.13 MHz for ¹H and 125.77 MHz for ¹³C. A 4 mm triple resonance probe with magic angle spinning (MAS) was used in all experiments. The samples were packed on a zirconia rotor and made to rotate at a MAS speed of 11-15 kHz. For the cross-polarization MAS experiments (13)C, 55 and 28 kHz RF fields were used for excitation and initial decoupling of protons, respectively. During the CP period, the RF (1)H field was increased by increments of 100, while the RF (13)C fields were maintained constantly. The protons were decoupled from the carbons during acquisition by using a phase modulation two-pulse decoupling method. A 62 kHz moderate ramp RF field was used for spin locking, while the carbon RF field was matched to obtain an optimal signal. The relaxation delay between the accumulations was 5 s and the contact time CP of 0.5 ms. All chemical shifts are reported in δ scale and refer externally to tetramethylsilane at 0 ppm.

### Results

### Molecular weight analysis

As illustrated in Figure 7A, the extract showed a molecular weight of over 2000 kDa, indicating that it resembles a high molecular weight variant of HA. On the contrary, Figure 7B reports the agarose gel relating to the Greenluronic^{®} samples dissolved with different SDS solutions. What can be observed is that, depending on the SDS solution, it was possible to reduce the molecular weight of Greenluronic^{®} differently; in fact, the 1% SDS solution reduced the molecular weight of Greenluronic^{®} from 4000 kDa to 2000 kDa, the 3% SDS solution reduced the molecular weight of Greenluronic^{®} from 4000 kDa to 1650 kDa, the 5% SDS solution reduced the molecular weight of Greenluronic^{®} from 4000 kDa to about 1000 kDa, and the 8% SDS solution reduced the molecular weight of Greenluronic^{®} from 4000 kDa to about 500 kDa.

### Polysaccharide spectrum by MALDI-TOF

By observing the MALDI-TOF spectra, it is possible to notice differences in the peak population. A more significant number of peaks characterizes the YEF sample than YET. The visible molecular weight difference between the high-intensity peaks is 162, corresponding to one unit of glucan. The smallest difference is 17 Da, which can be attributed to chlorine adducts or OH groups. A further interpretation of the MALDI-TOF spectra may be suggested by the fact that glucans were observed as sodium ions ([Glcn + Na]+) with a peak-to-peak mass difference of 162 Da, consistent with the repeat unit of b-(1 _xD83E__xDC6A_ 3)-glucan. Similar observations have been reported previously; Tremella's fruiting body is composed of 1→ 2)- and (1→ 4)-bound mannose and (1→ 3)-bound glucans.

### Chemical analysis and NMR

The data relating to the content of hyaluronic acid and polysaccharides in the samples are reported in Table 15 and the NMR spectrum was shown in Figure 8. From these results it emerged that all samples, both those with high molecular weight and those with low weight of Greenluronic^{®}, do not show significant differences in terms of hyaluronic acid and polysaccharides content

**Table 15 The data relating to the total polysaccharides and HA content were expressed in % w/w ± SD. For all analyses, three independent experiments were performed in quadruple.**

| **Analysis** | **Greenluronic^{®}** | **SDS Greenluronic^{®}** |
|---|---|---|
| Total polysaccharides (I.M.) | 90.6 ± 1.25 | 90.8 ± 1.70 |
| Total hyaluronic acid (I.M.) | 87.8 ± 1.43 | 87.4 ± 1.15 |

## Claims

1. An extract from *T.fuciformis* which comprises or consists of polysaccharides from *T.fuciformis* in an amount by weight comprised from 80% to 95% by weight, based on the total weight of the extract, wherein from 75% to 95% of said polysaccharides from *T.fuciformis* are polysaccharides identified as hyaluronic acid or a salt thereof having a weight average molecular weight comprised from 1000 kDa to 6000 kDa.

2. The extract according to claim 1, wherein said polysaccharides identified as hyaluronic acid or a salt thereof have a weight average molecular weight comprised from 1500 kDa to 5000 kDa, preferably comprised from 2000 kDa to 4000 kDa.

3. The extract according to claim 1 or 2, wherein said extract from *T.fuciformis* further comprises uronic acid in an amount by weight comprised from 1% to less than 3% by weight, preferably comprised from 1.5% to 2.5% by weight, more preferably comprised from 2% to 2.8% by weight, with respect to the total weight of the extract.

4. The extract according to any one of claims 1-3, wherein said extract from *T.Fuciformis* further comprises NAG in an amount by weight comprised from 0.01% to less than 0.1% by weight, preferably comprised from 0.04% to 0.08% by weight, with respect to the total weight of the extract.

5. The extract according to any one of claims 1-4, wherein said extract from *T.Fuciformis* further comprises water in an amount by weight comprised from 2% to 8%, preferably comprised from 4% to 6% by weight, with respect to the total weight of the extract.

6. The extract according to any one of claims 1-5, comprising or, alternatively, consisting of:
- polysaccharides from *T.fuciformis* in an amount by weight comprised from 80% to 95% by weight, with respect to the weight of the extract, wherein from 75% to 95% of said polysaccharides from *T.fuciformis* are polysaccharides identified as hyaluronic acid or a salt thereof having a weight average molecular weight comprised from 1000 kDa to 6000 kDa, preferably comprised from 1500 kDa to 5000 kDa, more preferably comprised from 2000 kDa to 4000 kDa;
- uronic acid in an amount by weight comprised from 1% to less than 3% by weight, preferably comprised from 1.5% to 2.5% by weight, more preferably comprised from 2% to 2.8% by weight, with respect to the total weight of the extract;
- NAG in an amount by weight comprised from 0.01% to less than 0.1% by weight, preferably comprised from 0.04% to 0.08% by weight, with respect to the total weight of the extract;
- water in an amount by weight comprised from 2% to 8%, preferably comprised from 4% to 6% by weight, with respect to the total weight of the extract,
wherein said extract is a liquid extract, preferably it is an aqueous liquid extract.

7. The extract according to any one of claims 1-6, wherein 1 gram of said liquid extract, added to 100 ml of ultrapure water at room temperature at 25 °C and under continuous stirring, has a pH value comprised from 5.5 to 7.5, preferably a pH value comprised from 6 to 7, more preferably 6.5.

8. A mixture which comprises or, alternatively, consists of said extract according to any one of claims 1-7, wherein said mixture is preferably in the form of powder and/or granules.

9. A composition which comprises or, alternatively, consists of:
- said mixture according to claim 8, and
- technological additives and/or excipients, of pharmaceutical or food grade.

10. The mixture according to claim 8 or the composition according to claim 9, for use as a medicament; preferably for use in a method of treatment, preventive or curative, for human beings and animals having diseases and/or disorders and/or symptoms chosen from the group comprising or, alternatively, consisting of arthritis, osteoarthritis, arthrosis, joint pain, inflammation of the limbs and joints, inflammation of cartilage, skin and epidermis.

11. A non-therapeutic use of the mixture according to claim 8 or of the composition according to claim 9, wherein said non-therapeutic use is a cosmetic or dermo-cosmetic use for counteracting ageing of the skin and epidermis.

12. A process for preparing an aqueous liquid extract comprising or, alternatively, consisting of a hyaluronic acid or a salt thereof, comprising the following steps:
(i) a step of identifying and selecting one or more natural fungi as a starting plant material of a hyaluronic acid or a salt thereof, wherein said natural fungi belong to the subkingdom *Dikarya,* preferably to the division *Basidiomycota,* more preferably they are of the species *Tremella fuciformis;*
(ii) optionally, a step of carrying out a crushing or pulverisation of the starting plant material;
(iii) a step of extracting said hyaluronic acid or salt thereof from the starting plant material obtained from step (i) or obtained from step (ii) by using an extraction solvent comprising or consisting of water, to give an aqueous extract of said hyaluronic acid or a salt thereof;
(iv) at least one step of enzymatic hydrolysis of said aqueous extract to give a hydrolysed aqueous extract;
(v) at least one concentration step; preferably, at least one step of vacuum concentration of said hydrolysed aqueous extract to give a concentrated aqueous extract;
(vi) at least one step of precipitation of said concentrated aqueous extract conducted by using a precipitation solution comprising or, alternatively, consisting of at least one alcohol, preferably ethanol, and/or water to give a precipitated aqueous extract;
(vii) at least one step of removing, from said precipitated aqueous extract, said precipitation solution comprising or, alternatively, consisting of at least one alcohol and/or water to give an intermediate aqueous extract;
(vii-1) at least one step of heat treating said intermediate aqueous extract to give an aqueous liquid extract.

13. The process according to claim 12, wherein the precipitation step (vi) comprises:
- a sub-step of removing part of the alcohol, preferably ethanol, contained in the precipitation solution,
and/or
- a sub-step of diluting the concentration of the alcohol, preferably ethanol, contained in the precipitation solution by adding water to the concentrated aqueous extract,
to allow the redissolution of hyaluronic acid or a salt thereof.

14. The process according to claim 12 or 13, wherein said step (vii-1) is a pasteurization heat treatment step.

15. The process according to any one of claims 12-14, further comprising a step (viii) of eliminating water from said aqueous liquid extract to give an extract in powder or granules, wherein said step (viii) is performed by dehydration or lyophilization.
